(19) Europäisches Patentamt
European Patent Office
Office européen des brevets

(11) Veröffentlichungsnummer : **0 054 970**
**B1**

(12) # EUROPÄISCHE PATENTSCHRIFT

(45) Veröffentlichungstag der Patentschrift : **17.07.85**

(51) Int. Cl.⁴ : **C 07 D501/24**, A 61 K 31/545

(21) Anmeldenummer : **81110739.0**

(22) Anmeldetag : **23.12.81**

(54) **7-Beta-(Ketooximinoacylamido)-ceph-3-em-4-carbonsäurederivate, Verfahren zur Herstellung derselben und diese enthaltende Arzneimittel.**

(30) Priorität : **24.12.80 IT 2694580**

(43) Veröffentlichungstag der Anmeldung : **30.06.82 Patentblatt 82/26**

(45) Bekanntmachung des Hinweises auf die Patenterteilung : **17.07.85 Patentblatt 85/29**

(84) Benannte Vertragsstaaten : **AT BE CH DE FR GB IT LI LU NL SE**

(56) Entgegenhaltungen :
**EP-A- 0 009 671**
**FR-A- 2 414 508**

(73) Patentinhaber : **I. S. F. Società per Azioni**
**Via Leonardo da Vinci, 1**
**I-20090 Trezzano s/N (Milano) (IT)**

(72) Erfinder : **Bellani, Piero**
**Via dei Mille 22211**
**Rho (Milano) (IT)**
Erfinder : **Bolis, Goffredo**
**Via Roma 49**
**Antegnate (IT)**
Erfinder : **Broccali, Giampietro, Dr.**
**Via Indipendenza 20**
**Milano (IT)**
Erfinder : **Giani, Roberto**
**Via Borsi 4**
**Milano (IT)**
Erfinder : **Pinza, Mario**
**Via per Cesano Boscone 32**
**Corsico (Milano) (IT)**

(74) Vertreter : **Beszédes, Stephan G. Dr.**
**Münchener Strasse 80a Postfach 1168**
**D-8060 Dachau (DE)**

**EP 0 054 970 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 7β-(Ketooximinoacylamido)-ceph-3-em-4-carbonsäurederivate, ein Verfahren zur Herstellung derselben sowie diese enthaltende Arzneimittel, insbesondere baktericide beziehungsweise antibakterielle Mittel.

Der Erfindung liegt die Aufgabe zugrunde, neue Cephalosporine mit überlegenen pharmakologischen, insbesondere baktericiden beziehungsweise antibakteriellen, Wirkungen, ein Verfahren zur Herstellung derselben und diese Verbindungen enthaltende Arzneimittel zu schaffen.

Das Obige wurde überraschenderweise durch die Erfindung erreicht.

Gegenstand der Erfindung sind 7β-(Ketooximinoacylamido)-ceph-3-em-4-carbonsäurederivate der allgemeinen Formel

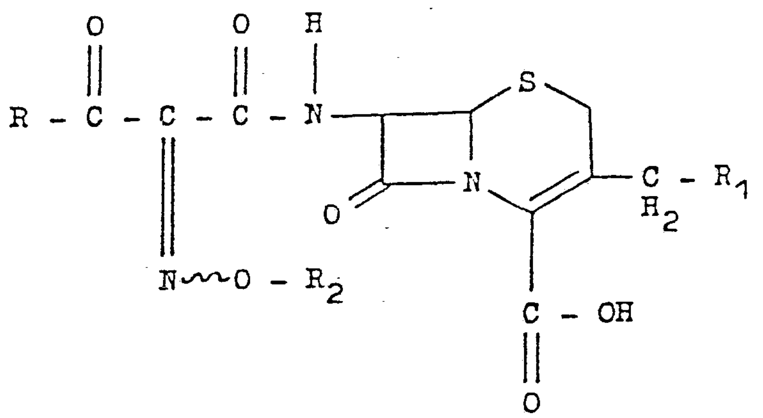

(I)

worin

R für einen Alkylrest mit 2 bis 4 Kohlenstoffatomen, einen Phenylrest oder einen, gegebenenfalls aminosubstituierten, als Heteroatom(e) Stickstoff, Sauerstoff und/oder Schwefel aufweisenden heterocyclischen Rest mit 5 Ringatomen steht,

$R_1$ einen Acetoxyrest, einen Carbamoyloxyrest oder einen Rest der allgemeinen Formel

$$—S—Het \qquad (II)$$

in welchletzterer

Het für einen, gegebenenfalls durch einen Alkyl- oder Sulfoalkylrest mit 1 bis 4 Kohlenstoffatom(en) oder eine Carboxylgruppe substituierten, heterocyclischen Rest mit 5 oder 6 Ringatomen und 1 oder mehr Stickstoffatom(en) und gegebenenfalls Schwefelatom(en) als Heteroatom(en) steht,

bedeutet,

$R_2$ Wasserstoff, einen Alkylrest mit 1 bis 4 Kohlenstoffatom(en) oder einen Acetylrest darstellt und die Wellenlinie das Vorliegen der syn- oder anti-Konfiguration oder von beiden derselben je nach der Konfiguration der Oximinogruppe bedeutet,

sowie ihre, zweckmäßig pharmazeutisch brauchbaren, Alkalimetallsalze.

Vorzugsweise ist der Alkylrest, für den R stehen kann, ein solcher mit 2 oder 3 Kohlenstoffatomen.

Es ist auch bevorzugt, daß der heterocyclische Rest, für den R stehen kann, ein Furylrest, ein Thienylrest oder ein, gegebenenfalls aminosubstituierter, Thiazolylrest ist.

Ferner ist es bevorzugt, daß der heterocyclische Rest, für welchen Het steht, ein Tetrazolyl-, Thiazolyl-, Thiadiazolyl- oder Pyridazinylrest ist.

Weiterhin ist es bevorzugt, daß der Alkyl- beziehungsweise Sulfoalkylrest, durch welchen der heterocyclische Rest, für den Het steht, substituiert sein kann, ein solcher mit 1 bis 3, insbesondere 1 oder 2, ganz besonders 1, Kohlenstoffatom(en) ist.

Vorzugsweise ist der Alkylrest, für den $R_2$ stehen kann, ein solcher mit 1 oder 2, insbesondere 1, Kohlenstoffatom(en).

Bevorzugte Alkalisalze der erfindungsgemäßen 7β-(Ketooximinoacylamido)-ceph-3-em-4-carbonsäurederivate der allgemeinen Formel I sind Natrium- und Kaliumsalze.

Besonders bevorzugte erfindungsgemäße Verbindungen sind 7β-(2'-Hydroxyimino-3'-oxovaleramido)-3-(acetoxymethyl)-ceph-3-em-4-carbonsäure, 7β-(2'-Hydroxyimino-3'-oxo-4'-methylvaleramido)-3-(acetoxymethyl)-ceph-3-em-4-carbonsäure und 7β-(2'-Hydroxyimino-3'-oxovaleramido)-3-[(1"-methyl-1"H-tetrazol-5"-yl)-thiomethyl]-ceph-3-em-4-carbonsäure.

Gegenstand der Erfindung ist auch ein Verfahren zur Herstellung der erfindungsgemäßen Verbindungen, welches dadurch gekennzeichnet ist, daß

a) 7β-Aminocephalosporansäure beziehungsweise Derivate derselben der allgemeinen Formel

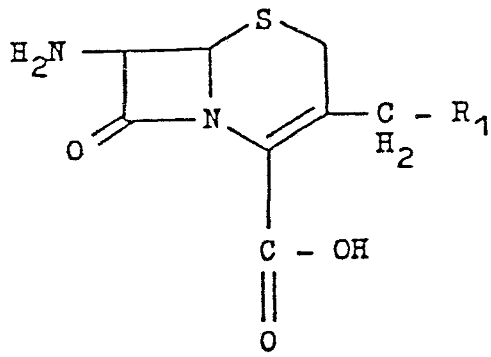

(III)

worin $R_1$ wie oben festgelegt ist, in geschützter Form in aprotonischen (aprotischen) Lösungsmitteln bei Temperaturen von 25 bis 100 °C mit 2,2-Dimethyl-4,6-dioxo-5-acyl-1,3-dioxanen der allgemeinen Formel

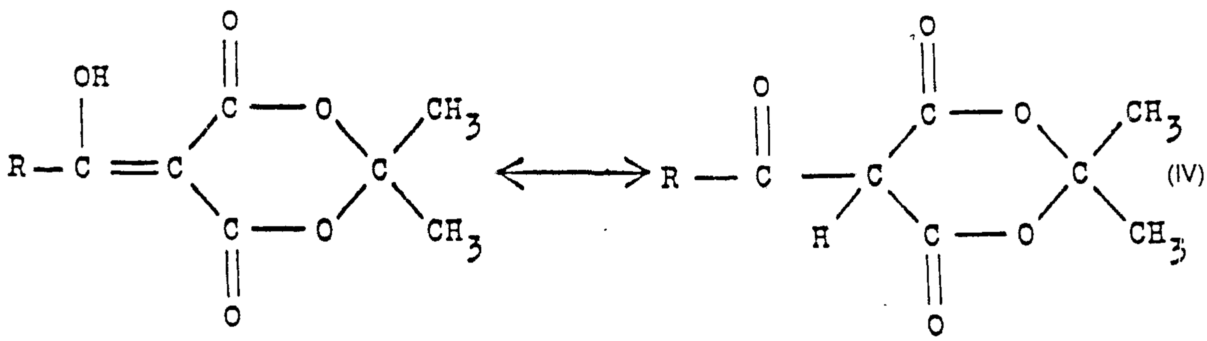

(IV)

(a) (b)

worin R wie oben festgelegt ist, zu 7β-(Ketoacylamido)-ceph-3-em-4-carbonsäurederivaten der allgemeinen Formel

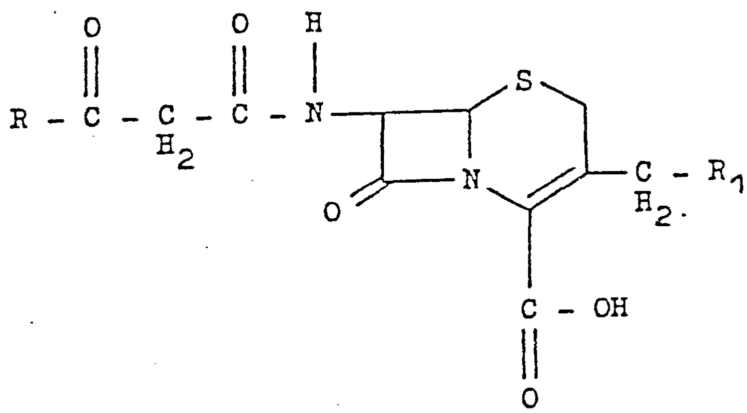

(V)

worin R und $R_1$ wie oben festgelegt sind, umgesetzt wird beziehungsweise werden und diese, gegebenenfalls in geschützter Form, in an sich bekannter Weise mit Lösungen von Alkalinitriten in Gegenwart von Essigsäure oder Ameisensäure zu 7β-(Ketooximinoacylamido)-ceph-3-em-4-carbonsäurederivaten der allgemeinen Formel I, bei welchen $R_2$ Wasserstoff bedeutet, nitrosiert werden und

α) gegebenenfalls diese, zweckmäßig in geschützter Form, in an sich bekannter Weise, alkyliert oder acetyliert werden und/oder

β) gegebenenfalls, soweit 7β-(Ketooximinoacylamido)-ceph-3-em-4-carbonsäurederivate der allgemeinen Formel I, bei welchen $R_1$ einen Acetoxyrest bedeutet, erhalten wurden, diese in an sich bekannter Weise in die entsprechenden 7β-(Ketooximinoacylamido)-ceph-3-em-4-carbonsäurederivate der allgemeinen Formel I, bei welchen $R_1$ einen Carbamoyloxyrest oder einen Rest der allgemeinen Formel II bedeutet, überführt werden oder

b) in an sich bekannter Weise 3-Oxo-2-alkoxyiminoalkansäuren beziehungsweise 3-Oxo-2-acetoxyiminoalkansäuren der allgemeinen Formel

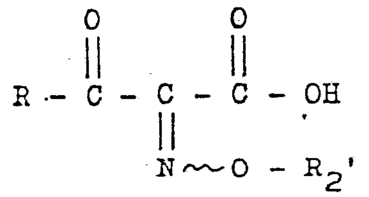

(VI)

worin $R_2'$ für einen Alkylrest mit 1 bis 4 Kohlenstoffatom(en) oder einen Acetylrest steht und R wie oben festgelegt ist, oder reaktionsfähige funktionelle Derivate derselben mit 7β-Aminocephalosporansäure beziehungsweise Derivaten derselben der allgemeinen Formel

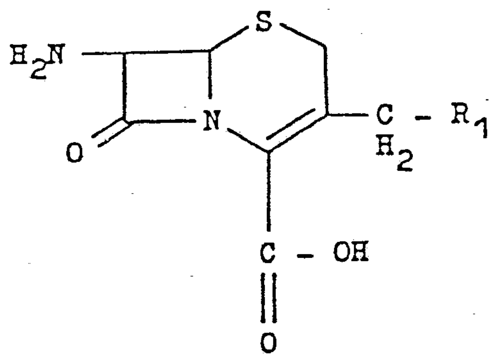

(III)

worin $R_1$ wie oben festgelegt ist, in geschützter Form zu 7β-(Ketooximinoacylamido)-ceph-3-em-4-carbonsäurederivaten der allgemeinen Formel I, bei welchen $R_2$ einen Alkylrest mit 1 bis 4 Kohlenstoffatom(en) oder einen Acetylrest bedeutet, umgesetzt werden,
worauf gegebenenfalls vorliegende Schutzgruppen in an sich bekannter Weise entfernt und/oder gegebenenfalls die erhaltenen 7β-(Ketooximinoacylamido)-ceph-3-em-4-carbonsäurederivate der allgemeinen Formel I in an sich bekannter Weise in Alkalisalze überführt werden.

Die im erfindungsgemäßen Verfahren als Ausgangsstoffe eingesetzten 2,2-Dimethyl-4,6-dioxo-5-acyl-1,3-dioxane der allgemeinen Formel IV existieren also in zueinander tantomeren Enol- und Ketoformen (a und b).

Die Umsetzung der 7β-Aminocephalosporansäure beziehungsweise Derivate derselben der allgemeinen Formel III mit den 2,2-Dimethyl-4,6-dioxo-5-acyl-1,3-dioxanen der allgemeinen Formel IV wird zweckmäßig in solchen aprotonischen Lösungsmitteln, welche einen nicht zu hohen Siedepunkt haben, durchgeführt. Als Lösungsmittel werden bevorzugt Tetrahydrofuran, Methylenchlorid beziehungsweise Chloroform verwendet.

Die an der Carboxylgruppe geschützte Form der im erfindungsgemäßen Verfahren als Ausgangsstoffe eingesetzten 7β-Aminocephalosphoransäure beziehungsweise Derivate derselben der allgemeinen Formel III ist vorteilhaft eine solche, welche mit N,O-bis-(Trimethylsilyl)-acetamid erhalten worden ist.

Vorteilhaft können als Alkalinitrite zum Nitrosieren der 7β-(Ketoacylamido)-ceph-3-em-4-carbonsäurederivate der allgemeinen Formel V Natrium- beziehungsweise Kaliumnitrit verwendet werden.

Ferner sind erfindungsgemäßen Arzneimittel, welche 1 oder mehr der erfindungsgemäßen Verbindungen als Wirkstoff beziehungsweise Wirkstoffe, zweckmäßig zusammen mit 1 oder mehr üblichen pharmazeutischen Konfektionierungsmittel(n) enthalten, vorgesehen.

Die erfindungsgemäßen Verbindungen haben nämlich wertvolle pharmakologische Eigenschaften. Insbesondere sind sie vorzügliche baktericide beziehungsweise antibakterielle Mittel. So haben sie eine starke antibakterielle Wirkung gegen gramnegative Mikroorganismen einschließlich β-Lactamase erzeugender Mikroorganismen. Sie haben auch eine wertvolle Wirksamkeit gegen grampositive Mikroorganismen.

Die antibakterielle Wirksamkeit « in vitro » wurde durch Bestimmen der Mindesthemmkonzentration in Bezug auf grampositive und gramnegative Mikroorganismen einschließlich β-Lactamase erzeugender Mikroorganismen in einem festen Agar-Kulturmedium (Isosensitest-Agar OXOID) ermittelt. Als bakterielles Impfmaterial wurde über eine Nacht bei 37 °C wachsen gelassene und zur Zeit des Gebrauches mit einem flüssigen Kulturmedium mit der Bezeichnung Difco vom Typ « brain heart infusion » (im Verhältnis von 1 : 25) verdünnte Kultur verwendet. Es wurden die Mindesthemmkonzentrationen in γ/cm³ von erfindungsgemäßen Verbindungen und der bekannten handelsüblichen (6R,7R)-3-(Carbamoyloxymethyl)-7-[(Z)-2-(2'-furyl)-2-(methoxyimino)-acetamido]-8-oxo-5-thia-1-azabicyclo-[4,2,0]oct-2-en-2-carbonsäure {Cefuroxim} mit anerkannt guter Wirkung gleicher Wirkungsrichtung bestimmt, wobei die Werte den Durchschnitt von 4 unter denselben Versuchsbedingungen durchgeführten Versuchen darstellen. Die Ergebnisse dieser Versuche sind in der folgenden Tabelle zusammengestellt.

(Siehe Tabelle 5 ff.)

Tabelle

| | Mikroorganismus | Mindesthemmkonzentrationen in $\gamma/cm^3$ von | | | |
|---|---|---|---|---|---|
| | | 7ß-(2'-Hydroxyimino-3'-oxovaleramido)-3-(acetoxymethyl)-ceph-3-em-4-carbonsäure {erfindungsgemäß, Beispiel 1} | 7ß-(2'-Hydroxyimino-3'-oxo-4'-methylvaleramido)-3-(acetoxymethyl)-ceph-3-em-4-carbonsäure {erfindungsgemäß, Beispiel 2} | 7ß-(2'-Hydroxyimino-3'-oxovaleramido)-3-[(1''-methyl-1''H-tetrazol-5''-yl)-thiomethyl]-ceph-3-em-4-carbonsäure {erfindungsgemäß, Beispiel 5} | (6R,7R)-3-(Carbamoyloxymethyl)-7-[(Z)-2-(2'-furyl)-2-(methoxyimino)-acetamido]-8-oxo-5-thia-1-azabicyclo[4,2,0]oct-2-en-2-carbonsäure ⟨Cefuroxim⟩ {Vergleichssubstanz} |
| grampositive | S. aureus ATCC 9144 | 0,39 | 0,19 | 0,13 | 0,62 |
| | S. aureus 6014668 (Penicillinase erzeugend) | 9,3 | 12,5 | 9,04 | 56,2 |
| | S. aureus F2 (Penicillinase erzeugend) | 6,25 | 6,25 | 9,04 | 24,3 |

Tabelle (Fortsetzung)

| | Mikroorganismus | Mindesthemmkonzentrationen in $\gamma/cm^3$ von | | | |
|---|---|---|---|---|---|
| | | 7ß-(2'-Hydroxyimino-3'-oxovaleramido)-3-(acetoxymethyl)-ceph-3-em-4-carbonsäure {erfindungsgemäß, Beispiel 1} | 7ß-(2'-Hydroxyimino-3'-oxo-4'-methylvaleramido)-3-(acetoxymethyl)-ceph-3-em-4-carbonsäure {erfindungsgemäß, Beispiel 2} | 7ß-(2'-Hydroxyimino-3'-oxovaleramido)-3-[1''-methyl-1''H-tetrazol-5''-yl)-thiomethyl]-ceph-3-em-4-carbonsäure {erfindungsgemäß, Beispiel 5} | (6R,7R)-3-(Carbamoyloxymethyl)-7-[(Z)-2-(2'-furyl)-2-(methoxyimino)-acetamido]-8-oxo-5-thia-1-azabicyclo[4,2,0]oct-2-en-2-carbonsäure ⟨Cefuroxim⟩ {Vergleichssubstanz} |
| grampositive | S. faecalis ATCC 6057 | 18,7 | 25 | 12,5 | 45 |
| | S. lutea ATCC 9341 | <0,012 | <0,012 | <0,012 | 0,056 |
| gramnegative | E. coli $R^+$ TEM (ß-Lactamase erzeugend) | 3,12 | - | 0,55 | 4,4 |
| | S. typhi 6/12 To | 0,39 | 1,56 | 0,067 | 1,56 |

Tabelle (Fortsetzung)

| Mikroorganismus | | Mindesthemmkonzentrationen in $\gamma/cm^3$ von | | | |
|---|---|---|---|---|---|
| | | 7ß-(2'-Hydroxyimino-3'-oxovaleramido)-3-(acetoxymethyl)-ceph-3-em-4-carbonsäure {erfindungsgemäß, Beispiel 1} | 7ß-(2'-Hydroxyimino-3'-oxo-4'-methylvaleramido)-3-(acetoxymethyl)-ceph-3-em-4-carbonsäure {erfindungsgemäß, Beispiel 2} | 7ß-(2'-Hydroxyimino-3'-oxovaleramido)-3-[(1''-methyl-1''H-tetrazol-5''-yl)-thiomethyl]-ceph-3-em-4-carbonsäure {erfindungsgemäß, Beispiel 5} | (6R,7R)-3-(Carbamoyloxymethyl)-7-[(Z)-2-(2'-furyl)-2-(methoxyimino)-acetamido]-8-oxo-5-thia-1-azabicyclo[4,2,0]oct-2-en-2-carbonsäure ⟨Cefuroxim⟩ {Vergleichssubstanz} |
| Gramnegative | S. typhimurium | 3,12 | 6,25 | 0,55 | 6,25 |
| | E. cloacae 214 (ß-Lactamase erzeugend) | 9,31 | 12,5 | 1,56 | 17,6 |
| | E. cloacae E 53 (ß-Lactamase erzeugend) | 9,31 | 12,5 | 2,2 | 17,6 |
| | S. paratyphi B | 3,12 | 6,25 | 0,27 | 8,83 |

Die erfindungsgemäßen Verbindungen sind selbst bei Dosen weit über den wirksamen Dosen praktisch nicht toxisch.

Die Erfindung wird an Hand der folgenden Beispiele näher erläutert.

Beispiel 1

7β-(2'-Hydroxyimino-3'-oxovaleramido)-3-(acetoxymethyl)-ceph-3-em-4-carbonsäure

Es wurde eine Suspension von 8,2 g 7β-Aminocephalosporansäure in einem Gemisch von 100 $cm^3$ Tetrahydrofuran und 24 $cm^3$ N,O-bis-(Trimethylsilyl)-acetamid unter Rückfluß zum Sieden erhitzt und 2 Stunden lang gerührt beziehungsweise geschüttelt, wodurch eine klare Lösung erhalten wurde. Dann wurden 6 g 2,2-Dimethyl-4,6-dioxo-5-propionyl-1,3-dioxan zugesetzt und die Mischung wurde 3 Stunden lang unter Erhitzen zum Sieden unter Rückfluß gerührt beziehungsweise geschüttelt und danach auf Raumtemperatur gekühlt und zur Trockne eingedampft. Der Rückstand wurde in 300 $cm^3$ Äthylacetat und 200 $cm^3$ Wasser gelöst. Das Unlösliche wurd abfiltriert und die Phasen wurden voneinander getrennt. Die organische Phase wurde mit 100 $cm^3$ Wasser gewaschen, getrocknet, mit Holzkohle entfärbt und zur Trockne eingedampft. Der Rückstand wurde in Diisopropyläther aufgenommen, bis eine gummiartige Konsistenz erhalten wurde, und dieses gummiartige Produkt wurde mit Diäthyläther verrieben. So wurden 2 g (18 % der Theorie) 7β-(3'-Oxovaleramido)-3-(acetoxymethyl)-ceph-3-emcarbonsäure mit einem Schmelzpunkt von 127 bis 134 °C (unter Zersetzung) erhalten.

Einer Lösung von 1,6 g der wie vorstehend beschrieben erhaltenen 7β-(3'-Oxovaleramido)-3-(acetoxymethyl)-ceph-3-em-4-carbonsäure in 25 $cm^3$ Eisessig wurde eine Lösung von 0,6 g Natriumnitrit in 6 $cm^3$ Wasser zugetropft.

Die Mischung wurde 1 1/2 Stunden lang gerührt beziehungsweise geschüttelt und dann wurden 100 $cm^3$ Äthylacetat und 100 $cm^3$ einer gesättigten Natriumchloridlösung zugesetzt. Die organische Phase wurde abgetrennt, 2-mal mit je 50 $cm^3$ Wasser gewaschen, wasserfrei gemacht und auf ein kleines Volumen eingeengt. Der Niederschlag wurde abfiltriert. So wurde 0,9 g (52 % der Theorie) 7β-(2'-Hydroxyimino-3'-oxovaleramido)-3-(acetoxymethyl)-ceph-3-em-4-carbonsäure mit einem Schmelzpunkt von 190 bis 204 °C (unter Zersetzung) und einem $[\alpha]_D^{25}$-Wert von + 97° (c = 0,2 in einem Gemisch von Tetrahydrofuran und Wasser in einem Volumverhältnis von 1 : 1) erhalten.

Beispiel 2

7β-(2'-Hydroxyimino-3'-oxo-4'-methylvaleramido)-3-(acetoxymethyl)-ceph-3-em-4-carbonsäure

Es wurde eine Suspension von 12,7 g 7β-Aminocephalosporansäure in einem Gemisch von 120 $cm^3$ Tetrahydrofuran und 37,5 $cm^3$ N,O-bis-(Trimethylsilyl)-acetamid 1 Stunde lang bei Raumtemperatur gerührt beziehungsweise geschüttelt, wodurch eine klare Lösung erhalten wurde. Zu dieser Mischung wurden 12,2 g 2,2-Dimethyl-4,6-dioxo-5-isobutyryl-1,3-dioxan zugegeben und es wurde 1 1/2 Stunden lang auf 60 °C erhitzt. Dann wurde das Gemisch auf Raumtemperatur gekühlt und zur Trockne eingedampft und der Rückstand in 300 $cm^3$ Äthylacetat und 200 $cm^3$ Wasser gelöst. Der unlösliche Rückstand wurde abfiltriert und die Phasen wurden getrennt. Die organische Phase wurde mit 50 $cm^3$ Wasser gewaschen, wasserfrei gemacht, mit Holzkohle entfärbt und auf ein kleines Volumen eingeengt. Dann wurde eine m Lösung des Natriumsalzes von 2-Äthylcapronsäure (2-ethyl sodium hexanoate) in Methylisobutylketon zugesetzt, bis ein pH-Wert von 8 erreicht wurde, worauf das Gemisch 20 Stunden lang unter Rühren beziehungsweise Schütteln stehengelassen und dann der Niederschlag abfiltriert wurde. So wurden 10,8 g (56,5 % der Theorie) des Natriumsalzes von 7β-(3'-Oxo-4'-methylvaleramido)-3-(acetoxymethyl)-ceph-3-em-4-carbonsäure mit einem Schmelzpunkt von 183 bis 185 °C (unter Zersetzung) erhalten.

Einer Lösung von 6,1 g des wie vorstehend beschrieben erhaltenen Natriumsalzes von 7β-(3'-Oxo-4'-methylvaleramido)-3-acetoxymethylceph-3-em-4-carbonsäure in 60 $cm^3$ Eisessig wurde eine Lösung von 2,1 g Natriumnitrit in 20 $cm^3$ Wasser zugetropft. Nach 2 Stunden langem Rühren beziehungsweise Schütteln bei Raumtemperatur wurden 200 $cm^3$ Äthylacetat und 100 $cm^3$ einer 0,1 n Salzsäure zugesetzt. Die Phasen wurden getrennt und die organische Phase wurde mit 50 $cm^3$ Wasser gewaschen, wasserfrei gemacht, mit Holzkohle entfärbt und auf ein kleines Volumen eingeengt. Der gefällte feste Stoff wurde abfiltriert. So wurden 1,4 g (22,6 % der Theorie) 7β-(2'-Hydroxyimino-3'-oxo-4'-methylvaleramido)-3-(acetoxymethyl)-ceph-3-em-4-carbonsäure mit einem Schmelzpunkt von 156 °C (unter Zersetzung) und einem $[\alpha]_D^{25}$-Wert von + 76,9° (c = 2 in einem Gemisch von Tetrahydrofuran und Wasser im Volumverhältnis von 1 : 1) erhalten.

Das als Ausgangssubstanz verwendete 2,2-Dimethyl-4,6-dioxo-5-isobutyryl-1,3-dioxan ist wie folgt hergestellt worden.

Es wurden zu einer Lösung von 14,4 g 2,2-Dimethyl-4,6-dioxo-1,3-dioxan in 150 $cm^3$ Methylenchlorid 19,4 $cm^3$ Pyridin zugegeben. Nach dem Kühlen auf 0 °C wurden 140 $cm^3$ einer 9 %-igen Lösung von Isobutyrylchlorid in Chloroform langsam zugegeben und dann wurde die Reaktionsmischung 1 Stunde lang bei 0 °C und dann noch 1 Stunde bei Raumtemperatur gerührt beziehungsweise geschüttelt. Sie

wurde zur Trockne eingedampft und der Rückstand wurde in 500 $cm^3$ Äthylacetat aufgenommen und 30 Minuten gerührt beziehungsweise geschüttelt, worauf filtriert und der gesammelte feste Stoff verworfen wurde. Das klare Filtrat wurde 2-mal mit einer 6,9 %-igen Lösung von Natriumbicarbonat in Wasser extrahiert und die wäßrigen Auszüge wurden gesammelt und mit 500 $cm^3$ Äthylacetat bedeckt und mit einer 20 %-igen Salzsäure auf einen pH-Wert von 1,5 angesäuert. Die Phasen wurden getrennt und die wäßrige Phase wurde erneut mit 100 $cm^3$ Äthylacetat extrahiert. Die vereinigten organischen Auszüge wurden mit 100 $cm^3$ einer gesättigten Natriumchloridlösung gewaschen und dann über Magnesiumsulfat getrocknet, mit Holzkohle entfärbt und zur Trockne eingedampft. So wurden 12 g (56 % der Theorie) 2,2-Dimethyl-4,6-dioxo-5-isobutyryl-1,3-dioxan in Form eines Öles erhalten.

Ultrarotspektrum (Film) : 1 745 $cm^{-1}$, 1 667 $cm^{-1}$ und 1 580 $cm^{-1}$.

Beispiel 3

7β-(2'-Hydroxyimino-3'-oxo-3'-phenylpropionamido)-3-(acetoxymethyl)-ceph-3-em-4-carbonsäure

Es wurden 5 g 7-Aminocephalosporansäure, 15 $cm^3$ N,O-bis-(trimethylsilyl)-acetamid und 100 $cm^3$ Tetrahydrofuran 45 Minuten lang bei Raumtemperatur gerührt beziehungsweise geschüttelt und zur Mischung wurden 5 g 2,2-Dimethyl-4,6-dioxo-5-benzoyl-1,3-dioxan zugegeben. Nach 1 Stunde und 20 Minuten wurde unter Vakuum zur Trockne eingedampft, der Rückstand wurde in 100 $cm^3$ Äthylacetat aufgenommen und die erhaltene Lösung wurde 15 Minuten lang mit 20 $cm^3$ Wasser gerührt beziehungsweise geschüttelt. Die organische Phase wurde abgetrennt, über wasserfreiem Magnesiumsulfat wasserfrei gemacht und unter Vakuum auf ein geringes Volumen eingeengt. Es fiel ein weißer kristalliner fester Stoff aus, welcher abfiltriert wurde. So wurden 6,35 g (82,8 % der Theorie) 7β-(3'-Oxo-3'-phenylpropionamido)-3-(acetoxymethyl)-ceph-3-em-4-carbonsäure mit einem Schmelzpunkt von 173 bis 174 °C (unter Zersetzung) und einem $[\alpha]_D^{25}$-Wert von + 63,9° (c = 1 in Tetrahydrofuran) erhalten.

Einer Lösung von 4,8 g der wie vorstehend beschrieben erhaltenen 7β-(3'-Oxo-3'-phenylpropionamido)-3-(acetoxymethyl)-ceph-3-em-4-carbonsäure in 115 $cm^3$ Essigsäure wurde während 10 Minuten eine Lösung von 1,58 g Natriumnitrit in 11,5 $cm^3$ Wasser zugetropft. Nach 1 Stunde langem Rühren beziehungsweise Schütteln wurde die Mischung in 500 $cm^3$ Äthylacetat eingegossen. Die so erhaltene Lösung wurde 4-mal mit je 100 $cm^3$ Wasser gewaschen, über wasserfreiem Magnesiumsulfat wasserfrei gemacht und unter Vakuum zur Trockne eingedampft. Der Rückstand wurde in Isopropyläther aufgeschlämmt und abfiltriert. So wurden 5,1 g (99 % der Theorie) 7β-(2'-Hydroxyimino-3'-oxo-3'-phenylpropionamido)-3-(acetoxymethyl)-ceph-3-em-4-carbonsäure mit einem Schmelzpunkt von 115 bis 135 °C (unter Zersetzung) und einem $[\alpha]_D^{25}$-Wert von + 41,30° (c = 1 in Tetrahydrofuran) erhalten.

Das als Ausgangssubstanz verwendete 2,2-Dimethyl-4,6-dioxo-5-benzoyl-1,3-dioxan ist wie folgt hergestellt worden.

Es wurden 14,4 g 2,2-Dimethyl-4,6-dioxo-1,3-dioxan in 200 $cm^3$ wasserfreiem Tetrahydrofuran und 11 g 50 %-iges Natriumhydrid in Mineralöl 1 Stunde lang gerührt beziehungsweise geschüttelt, worauf eine Lösung von 15 $cm^3$ Benzoylchlorid in 50 $cm^3$ wasserfreiem Tetrahydrofuran zugetropft wurde. Nach 16 Stunden langem Rühren beziehungsweise Schütteln wurde unter Vakuum zur Trockne eingedampft und der Rückstand wurde in 200 $cm^3$ Diäthyläther aufgenommen und abfiltriert. Er wurde in 100 $cm^3$ Wasser gelöst und die geringe Menge unlösliches Material wurde von der Lösung abfiltriert. Die wäßrige Lösung wurde mit einer 10 %-igen Salzsäure angesäuert und mit 100 $cm^3$ Äthylacetat extrahiert. Die organische Phase wurde über wasserfreiem Magnesiumsulfat wasserfrei gemacht und unter Vakuum zur Trockne eingedampft. Der Rückstand wurde in Diäthyläther aufgeschlämmt und abfiltriert. So wurden 11,8 (47,5 % der Theorie) 2,2-Dimethyl-4,6-dioxo-5-benzoyl-1,3-dioxan mit einem Schmelzpunkt von 107 bis 108 °C (unter Zersetzung) erhalten.

Beispiel 4

7β-[2'-Hydroxyimino-3'-oxo-3'-(fur-2''-yl)-propionamido]-3-(acetoxymethyl)-ceph-3-em-4-carbonsäure

Es wurde analog wie im Beispiel 3 beschrieben gearbeitet, jedoch als Ausgangssubstanz 2,2-Dimethyl-4,6-dioxo-5-(fur-2'-oyl)-1,3-dioxan verwendet. So wurden zunächst 7β-[3'-Oxo-3'-(fur-2''-yl)-propionamido]-3-(acetoxymethyl)-ceph-3-em-4-carbonsäure mit einem Schmelzpunkt von 170 bis 171 °C in einer Ausbeute von 76 % der Theorie und dann 7β-[2'-Hydroxyimino-3'-oxo-3'-(fur-2''-yl)-propionamido]-3-(acetoxymethyl)-ceph-3-em-4-carbonsäure mit einem Schmelzpunkt über 300 °C (unter Zersetzung) und einem $[\alpha]_D^{25}$-Wert von + 43,5° (c = 1 in Aceton) in einer Ausbeute von 77 % der Theorie erhalten.

Das als Ausgangssubstanz verwendete 2,2-Dimethyl-4,6-dioxo-5-(fur-2'-oyl)-1,3-dioxan ist analog wie die Ausgangssubstanz des Beispieles 3, jedoch unter Verwendung von Fur-2-oylchlorid an Stelle des Isobutyrylchlorides erhalten worden.

Beispiel 5

7β-(2'-Hydroxyimino-3'-oxovaleramido)-3-[(1''-methyl-1''H-tetrazol-5''-yl)-thiomethyl]-ceph-3-em-4-carbonsäure

Es wurde eine Suspension von 3,3 g 7β-Amino-3-[(1'-methyl-1'H-tetrazol-5'-yl)-thiomethyl]-ceph-3-em-4-carbonsäure in einem Gemisch von 50 $cm^3$ Tetrahydrofuran und 8 $cm^3$ N,O-bis-(Trimethylsilyl)-acetamid 30 Minuten lang gerührt beziehungsweise geschüttelt und dann wurden 2 g 2,2-Dimethyl-4,6-dioxo-5-propionyl-1,3-dioxan zugesetzt, worauf die Mischung 24 Stunden lang gerührt beziehungsweise geschüttelt und zur Trockne eingedampft und der Rückstand in 200 $cm^3$ Äthylacetat und 150 $cm^3$ einer 5 %-igen Salzsäure aufgenommen wurde. Nach 15 Minuten langem Rühren beziehungsweise Schütteln wurde das Unlösliche abfiltriert und die organische Phase wurde abgetrennt und mit 50 $cm^3$ Wasser gewaschen, wasserfrei gemacht, mit Holzkohle entfärbt und auf ein geringes Volumen eingeengt. Es wurden 50 $cm^3$ Isopropylalkohol und einem Lösung des Natriumsalzes der 2-Äthylcapronsäure in Isopropylalkohol bis zur Erreichung einer schwach alkalischen Reaktion zugesetzt. Die Mischung wurde 20 Stunden lang stehengelassen, worauf der Niederschlag abfiltriert wurde. So wurden 1,7 g (38 % der Theorie) des Natriumsalzes von 7β-(3'-Oxovaleramido)-3-[(1"-methyl-1"H-tetrazol-5"-yl)-thiomethyl]-ceph-3-em-4-carbonsäure mit einem Schmelzpunkt von 144 bis 145,5 °C (unter Zersetzung) erhalten.

Zu einer Lösung von 1,7 g des wie vorstehend beschrieben erhaltenen Natriumsalzes von 7β-(3'-Oxovaleramido)-3-[(1"-methyl-1"H-tetrazol-5"-yl)-thiomethyl]-ceph-3-em-4-carbonsäure in 20 $cm^3$ Eisessig wurde eine Lösung von 0,5 g Natriumnitrit in 5 $cm^3$ Wasser zugegeben. Nach 1 1/2 Stunden langem Rühren beziehungsweise Schütteln bei Raumtemperatur wurden zur Mischung 100 $cm^3$ Äthylacetat und 50 $cm^3$ einer 5 %-igen Salzsäure zugegeben. Die Phasen wurden getrennt und die organische Phase wurde mit 50 $cm^3$ Wasser gewaschen, wasserfrei gemacht, mit Holzkohle entfärbt und zur Trockne eingedampft. Der Rückstand wurde mit Diäthyläther verrieben. So wurde 0,7 g (40,5 % der Theorie) 7β-(2'-Hydroxyimino-3'-oxovaleramido)-3-[(1'methyl-1"H-tetrazol-5"-yl)-thiomethyl]-ceph-3-em-4-carbonsäure mit einem Schmelzpunkt von 79 bis 87 °C (unter Zersetzung) und einem $[\alpha]_D^{25}$-Wert von − 25° (c = 0,2 in einem Gemisch von Tetrahydrofuran und Wasser im Volumverhältnis von 1 : 1) erhalten.

## Beispiel 6

7β-(2'-Hydroxyimino-3'-oxovaleramido)-3-[(1"-{sulfomethyl}-1"H-tetrazol-5"-yl)-thiomethyl]-ceph-3-em-4-carbonsäure

Es wurde eine Mischung von 0,82 g wie im Beispiel 1 beschrieben erhaltener 7β-(2'-Hydroxyimino-3'-oxovaleramido)-3-(acetoxymethyl)-ceph-3-em-4-carbonsäure, 0,34 g Natriumbicarbonat und 0,4 g des Dinatriumsalzes von 1-(Sulfomethyl)-1H-tetrazol-5-thiol in 30 $cm^3$ eines Phosphatpuffers von einem pH-Wert von 6,5 4 Stunden lang auf 60 °C erhitzt. Es wurde auf 0 °C gekühlt und mit einer 10 %-igen Salzsäure auf einen pH-Wert von 1 angesäuert. Dann wurde filtriert und das Filtrat wurde mit Äthylacetat gewaschen und unter Vakuum zur Trockne eingedampft. Der Rückstand wurde in Methylalkohol aufgenommen, das Unlösliche wurde abfiltriert und das Filtrat wurde zur Trockne eingedampft. Der Rückstand wurde in einem Gemisch von Äthylalkohol und Acetonitril verrieben. So wurde 0,2 g (18,2 % der Theorie) 7β-(2'-Hydroxyimino-3'-oxovaleramido)-3-[(1"-{sulfomethyl}-1"H-tetrazol-5"-yl)-thiomethyl]-ceph-3-em-4-carbonsäure mit einem Schmelzpunkt über 145 °C (unter Zersetzung) und einem $[\alpha]_D^{25}$-Wert von + 8,7° (c = 0,5 in einem Phosphatpuffer mit einem pH-Wert von 7,4) erhalten.

## Beispiel 7

7β-(2'-Hydroxyimino-3'-oxovaleramido)-3-[(3"-carboxypyridazin-6"-yl)-thiomethyl]-ceph-3-em-4-carbonsäure

Es wurde wie im Beispiel 6 beschrieben gearbeitet, jedoch als Ausgangssubstanz 0,26 g 6-Mercaptopyridazin-3-carbonsäure statt des 1-(Sulfomethyl)-1H-tetrazol-5-thioles verwendet. So wurde 0,11 g (13,3 % der Theorie) 7β-(2'-Hydroxyimino-3'-oxovaleramido)-3-[(3"-carboxypyridazin-6"-yl)-thiomethyl]-ceph-3-em-4-carbonsäure mit einem Schmelzpunkt über 170 °C (unter Zersetzung) und einem $[\alpha]_D^{25}$-Wert von + 18,8° (c = 1 in einem Phosphatpuffer mit einem pH-Wert von 7,0) erhalten.

## Beispiel 8

7β-(2'-Hydroxyimino-3'-oxovaleramido)-3-(carbamoyloxymethyl)-ceph-3-em-4-carbonsäure

Es wurden zu einer Suspension von 7β-Amino-3-(carbamoyloxymethyl)-ceph-3-em-4-carbonsäure in 40 $cm^3$ Tetrahydrofuran 8 $cm^3$ N,O-bis-(Trimethylsilyl)-acetamid zugegeben. Die Mischung wurde 2 Stunden lang unter Rückfluß zum Sieden erhitzt und zur so erhaltenen klaren Lösung wurden 2 g 2,2-Dimethyl-4,6-dioxo-5-propionyl-1,3-dioxan zugegeben. Es wurde 2 Stunden lang unter Rühren beziehungsweise Schütteln und unter Rückfluß zum Sieden erhitzt und dann zur Trockne eingedampft. Der Rückstand wurde in 150 $cm^3$ Äthylacetat gelöst und zur erhaltenen Lösung wurden 50 $cm^3$ einer 5 %-igen Salzsäure zugegeben und es wurde noch 15 Minuten gerührt beziehungsweise geschüttelt. Die Verunreinigungen wurden abfiltriert und die organische Phase wurde abgetrennt und mit 50 $cm^3$ Wasser

gewaschen, über wasserfreiem Magnesiumsulfat wasserfrei gemacht, mit Holzkohle entfärbt und zur Trockne eingedampft. Der Rückstand wurde in Diäthyläther verrieben und aus Äthylacetat umkristallisiert. So wurde 0,65 g (17,5 % der Theorie) 7β-(3'-Oxovaleramido)-3-(carbamoyloxymethyl)-ceph-3-em-4-carbonsäure mit einem Schmelzpunkt von 152 °C (unter Zersetzung) erhalten.

Zu einer Lösung von 1 g wie vorstehend beschrieben erhaltener 7β-(3'-Oxovaleramido)-3-(carbamoyloxymethyl)-ceph-3-em-4-carbonsäure in 25 $cm^3$ einer 70 %-igen Essigsäure wurde während 30 Minuten eine Lösung von 0,37 g Natriumnitrit in 5 $cm^3$ Wasser zugegeben. Nach 2 Stunden langem Rühren beziehungsweise Schütteln bei Raumtemperatur wurden 100 $cm^3$ Äthylacetat zugesetzt. Die Lösung wurde mit 50 $cm^3$ einer gesättigten Natriumchloridlösung und dann mit 50 $cm^3$ Wasser gewaschen. Die organische Phase wurde über wasserfreiem Magnesiumsulfat getrocknet, mit Holzkohle entfärbt und zur Trockne eingedampft. Der Rückstand wurde in Diäthyläther verrieben. So wurde 0,45 g (41,7 % der Theorie) 7β-(2'-Hydroxyimino-3'-oxovaleramido)-3-(carbamoyloxymethyl)-ceph-3-em-4-carbonsäure mit einem Schmelzpunkt von 151 bis 157 °C (unter Zersetzung) erhalten.

## Beispiel 9

7β-(2'-Hydroxyimino-3'-oxovaleramido)-3-[(5"-methyl-1",3",4"-thiadiazol-2"-yl)-thiomethyl]-ceph-3-em-4-carbonsäure

Es wurden zu einer Suspension von 3,4 g 7β-Amino-3-[(5'-methyl-1',3',4'-thiadiazol-2'-yl)-thiomethyl]-ceph-3-em-4-carbonsäure in 50 $cm^3$ Tetrahydrofuran 8 $cm^3$ N,O-bis-(Trimethylsilyl)-acetamid zugegeben. Es wurde 2 Stunden lang unter Rückfluß zum Sieden erhitzt und zur erhaltenen klaren Lösung wurden 2 g 2,2-Dimethyl-4,6-dioxo-5-propionyl-1,3-dioxan zugegeben. Die Mischung wurde 2 Stunden lang unter Rühren beziehungsweise Schütteln und unter Rückfluß zum Sieden erhitzt und dann unter Vakuum zur Trockne eingedampft. Der Rückstand wurde in 150 $cm^3$ Äthylacetat gelöst und zur erhaltenen Lösung wurden 50 $cm^3$ einer 5 %-igen Salzsäure zugegeben. Nach 15 Minuten langem Rühren beziehungsweise Schütteln wurden die Verunreinigungen abfiltriert und vom Filtrat wurde die organische Phase abgetrennt und sie wurde mit 50 $cm^3$ Wasser gewaschen, über wasserfreiem Magnesiumsulfat wasserfrei gemacht, mit Holzkohle entfärbt und auf ein geringes Volumen eingeengt. Der erhaltene kristalline feste Stoff wurde abfiltriert und getrocknet. So wurden 1,5 g (34 % der Theorie) 7β-(3'-Oxovaleramido)-3-[(5"-methyl-1",3",4"-thiadiazol-2"-yl)-thiomethyl]-ceph-3-em-4-carbonsäure mit einem Schmelzpunkt von 146 bis 149 °C (unter Zersetzung) erhalten.

Zu einer Lösung von 1,1 g der wie vorstehend beschrieben erhaltenen 7β-(3'-Oxovaleramido)-3-[(5"-methyl-1",3",4"-thiadiazol-2"-yl)-thiomethyl]-ceph-3-em-4-carbonsäure in 25 $cm^3$ einer 60 %-igen Essigsäure wurde während 30 Minuten eine Lösung von 0,35 g Natriumnitrit in 50 $cm^3$ Wasser zugegeben. Nach 2 Stunden langem Rühren beziehungsweise Schütteln bei Raumtemperatur wurden 100 $cm^3$ Äthylacetat zugegeben und die Lösung wurde mit 50 $cm^3$ einer gesättigten Natriumchloridlösung und mit 50 $cm^3$ Wasser gewaschen. Die organische Phase wurde über wasserfreiem Magnesiumsulfat wasserfrei gemacht, mit Holzkohle entfärbt und zur Trockne eingedampft. Der Rückstand wurde in Diäthyläther verrieben. So wurde 0,5 g (42,4 % der Theorie) 7β-(2'-Hydroxyimino-3'-oxovaleramido)-3-[(5"-methyl-1",3",4"-thiadiazol-2"-yl)-thiomethyl]-ceph-3-em-4-carbonsäure mit einem Schmelzpunkt von 118 bis 119 °C (unter Zersetzung) erhalten.

## Beispiel 10

7β-(2'-Acetoxyimino-3'-oxovaleramido)-3-(acetoxymethyl)-ceph-3-em-4-carbonsäure

Es wurden zu einer Lösung von 3 g wie im Beispiel 1 beschrieben erhaltener 7β-(2'-Hydroxyimino-3'-oxovaleramido)-3-(acetoxymethyl)-ceph-3-em-4-carbonsäure in 50 $cm^3$ Tetrahydrofuran 1,42 $cm^3$ Essigsäureanhydrid zugegeben. Nach 3 Stunden langem Rühren beziehungsweise Schütteln bei Raumtemperatur wurde die Mischung zur Trockne eingedampft und der Rückstand wurde aus Methylenchlorid umkristallisiert. So wurden 2,1 g (63,5 % der Theorie) 7β-(2'-Acetoxyimino-3'-oxovaleramido)-3-(acetoxymethyl)-ceph-3-em-4-carbonsäure mit einem Schmelzpunkt von 161 °C (unter Zersetzung) erhalten.

## Beispiel 11

7β-(2'-Methoxyimino-3'-oxovaleramido)-3-(acetoxymethyl)-ceph-3-em-4-carbonsäure

Es wurde einer Lösung von 5 g wie im Beispiel 1 beschrieben erhaltener 7β-(3'-Oxovaleramido)-3-(acetoxymethyl)-ceph-3-em-4-carbonsäure in 100 $cm^3$ Tetrahydrofuran eine Lösung von 3,18 g Diphenyldiazomethan in 50 $cm^3$ Tetrahydrofuran zugetropft und die Mischung wurde 5 Stunden lang bei Raumtemperatur gerührt beziehungsweise geschüttelt. Nach dem Eindampfen zur Trockne unter Vakuum wurde der feste Stoff in n-Hexan verrieben und abfiltriert und danach in Diäthyläther verrieben und erneut abfiltriert. So wurden 4,4 g (60,8 % der Theorie) des Benzhydrylesters von 7β-(3'-Oxovalerami-

do)-3-(acetoxymethyl)-ceph-3-em-4-carbonsäure mit einem Schmelzpunkt von 105,8 bis 107,1 °C erhalten.

Einer Lösung von 6 g wie vorstehend beschrieben erhaltenem Benzhydrylester von 7β-(3'-Oxovaleramido)-3-acetoxymethyl)-ceph-3-em-4-carbonsäure in 20 $cm^3$ Eisessig wurde eine Lösung von 1,54 g Natriumnitrit in 15 $cm^3$ Wasser zugetropft und die Mischung wurde 2 Stunden lang bei Raumtemperatur gerührt beziehungsweise geschüttelt. Dann wurde die Mischung mit 500 $cm^3$ Äthylacetat verdünnt und 3-mal mit je 100 $cm^3$ einer wäßrigen gesättigten Natriumchloridlösung gewaschen. Die organische Phase wurde wasserfrei gemacht und zur Trockne eingedampft und der Rückstand wurde in Diäthyläther verrieben. So wurden 5,5 g (86,6 % der Theorie) des Benzhydrylesters von 7β-(2'-Hydroxyimino-3'-oxovaleramido)-3-(acetoxymethyl)-ceph-3-em-4-carbonsäure mit einem Schmelzpunkt von 56 bis 62 °C erhalten.

Zu einer Lösung von 16 g wie vorstehend beschrieben erhaltenem Benzhydrylester von 7β-(2'-Hydroxyimino-3'-oxovaleramido)-3-(acetoxymethyl)-ceph-3-em-4-carbonsäure in 175 $cm^3$ Tetrahydrofuran wurde eine Lösung von 1,75 g Diazomethan in 100 $cm^3$ Diäthyläther zugegeben. Die Mischung wurde 30 Minuten lang bei Raumtemperatur gerührt beziehungsweise geschüttelt und zur Trockne eingedampft. Der Rückstand wurde in Diäthyläther verrieben und chromatographiert (Silicagel, Gemisch von n-Hexan und Äthylacetat im Volumverhältnis von 6 : 4). So wurden 3 g (18,3 % der Theorie) Benzhydrylester von 7β-(2'-Methoxyimino-3'-oxovaleramido)-3-(acetoxymethyl)-ceph-3-em-4-carbonsäure mit einem Schmelzpunkt von 164 bis 168 °C erhalten.

Einer Lösung von 2 g des wie vorstehend beschrieben erhaltenen Benzhydrylesters von 7β-(2'-Methoxyimino-3'-oxovaleramido)-3-(acetoxymethyl)-ceph-3-em-4-carbonsäure in 10 $cm^3$ Anisol wurden unter Kühlen auf 0 °C 8 $cm^3$ Trifluoressigsäure zugetropft. Die Reaktionsmischung wurde 30 Minuten lang bei 0 °C gerührt beziehungsweise geschüttelt, dann wurden 200 $cm^3$ Diäthyläther zugesetzt und es wurde 1 Stunde bei Raumtemperatur gerührt beziehungsweise geschüttelt. Der feste Stoff wurde unter Vakuum gesammelt. So wurde 0,9 g (63 % der Theorie) 7β-(2'-Methoxyimino-3'-oxovaleramido)-3-(acetoxymethyl)-ceph-3-em-4-carbonsäure mit einem Schmelzpunkt von 175,7 bis 181 °C erhalten.

## Beispiel 12

7β-(2'-Methoxyimino-3'-oxovaleramido)-3-[(1"-methyl-1"H-tetrazol-5"-yl)-thiomethyl]-ceph-3-em-4-carbonsäure

Es wurden einer Lösung von 7,5 g wie im Beispiel 5 beschrieben erhaltener 7β-(2'-Hydroxyimino-3'-oxovaleramido)-3-[(1"-methyl-1"H-tetrazol-5"-yl)-thiomethyl]-ceph-3-em-4-carbonsäure in einem Gemisch von 100 $cm^3$ Aceton und 100 $cm^3$ Wasser unter Kühlen auf 0 °C portionsweise 5 g Natriumcarbonat zugesetzt und dann 7,4 $cm^3$ Dimethylsulfat zugetropft. Dann wurde 30 Minuten lang gerührt beziehungsweise geschüttelt und danach wurden noch 5 g Natriumbicarbonat und 3,7 $cm^3$ Dimethylsulfat zugesetzt. Das Rühren beziehungsweise Schütteln wurde 1 Stunde fortgesetzt, es wurden 350 $cm^3$ Wasser zugesetzt, der pH-Wert wurde mit einer 10 %-igen Salzsäure auf 2 eingestellt und die wäßrige Schicht wurde mit Äthylacetat extrahiert und der Äthylacetatauszug wurde mit Wasser gewaschen, wasserfrei gemacht und zur Trockne eingedampft. Der so erhaltene ölige Rückstand wurde mehrere Male mit n-Hexan gewaschen, in 100 $cm^3$ Diäthyläther verrieben, unter Vakuum abgetrennt und mit 125 $cm^3$ Äthylacetat extrahiert. Das unlösliche wurde entfernt und das klare Filtrat wurde unter Vakuum zur Trockne eingedampft und der Rückstand wurde in Diäthyläther verrieben und unter Vakuum abgetrennt. So wurden 1,7 g (22 % der Theorie) 7β-(2'-Methoxyimino-3'-oxovaleramido)-3-[(1"-methyl-1"H-tetrazol-5"-yl)-thiomethyl]-ceph-3-em-4-carbonsäure mit einem Schmelzpunkt über 67 °C (unter Zersetzung) und einem $[\alpha]_D^{25}$-Wert von − 38,3° (c = 0,2 in einem Gemisch von Tetrahydrofuran und Wasser im Volumverhältnis von 1 : 1) erhalten.

## Beispiel 13

7β-[2'-Hydroxyimino-3'-oxo-3'-(2"-aminothiazol-4"-yl)-propionamido]-3-(acetoxymethyl)-ceph-3-em-4-carbonsäure

Es wurde zu einer Suspension von 2,3 g 2,2-Dimethyl-5-[1'-hydroxy-1'-(2"-{tert.-butoxycarbonylamino}-thiazol-4"-yl)-methyliden]-4,6-dioxo-1,3-dioxan in 30 $cm^3$ wasserfreiem Dioxan eine Lösung, welche durch 5 Minuten langes Erhitzen von 1,53 g 7β-Aminocephalosporansäure, 4,61 $cm^3$ N,O-bis-(Trimethylsilyl)-acetamid und 75 $cm^3$ wasserfreiem Dioxan zum Sieden unter Rückfluß erhalten worden ist, zugegeben, das Gemisch wurde 15 Minuten lang zum Sieden erhitzt, das Lösungsmittel wurde unter Vakuum abgedampft und der Rückstand wurde in ein Gemisch von 50 $cm^3$ Äthylacetat und 30 $cm^3$ einer n-Salzsäure aufgenommen. Das Gemisch wurde 10 Minuten lang gerührt beziehungsweise geschüttelt, die Phasen wurden getrennt und die organische Phase wurde mit 10 $cm^3$ einer gesättigten Natriumchloridlösung gewaschen und dann über wasserfreiem Magnesiumsulfat wasserfrei gemacht. Das Lösungsmittel wurde unter Vakuum abgedampft und der Rückstand wurde in Diäthyläther aufgenommen und unter Vakuum filtriert. Es wurden 1,25 g (37,3 % der Theorie) 7β-[3'-Oxo-3'-(2"-{tert.-butoxycarbonyla-

mino}-thiazol-4"-yl)-propionamido]-3-(acetoxymethyl)-ceph-3-em-4-carbonsäure mit einem $R_f$-Wert von 0,31 bei der Dünnschichtchromatographie [Silicagel F 254 ; Eluiermittel : Gemisch von 1,2-Dichloräthan und Methanol im Volumverhältnis von 7 : 3] erhalten.

Zu einer Lösung von 1,20 g der wie vorstehend beschrieben erhaltenen 7β-[3'-Oxo-3'-(2"-{tert.-butoxycarbonylamino}-thiazol-4"-yl)-propoionamido]-3-(acetoxymethyl)-ceph-3-em-4-carbonsäure in 24 $cm^3$ Eisessig wurde eine Lösung von 0,30 g Natriumnitrit in 24 $cm^3$ Wasser zugegeben. Die Mischung wurde 30 Minuten lang bei Raumtemperatur gerührt beziehungsweise geschüttelt, das Lösungsmittel wurde unter Vakuum abgedampft, der Rückstand wurde in ein Gemisch aus 120 $cm^3$ Äthylacetat und 60 $cm^3$ einer 0,1 n Salzsäure aufgenommen, die Schichten wurden voneinander getrennt und die organische Schicht wurde über wasserfreiem Magnesiumsulfat wasserfrei gemacht und unter Vakuum zur Trockne eingedampft. Der Rückstand wurde mit Isopropyläther verrieben und abfiltriert. Es wurden 1,20 g (95 % der Theorie) 7β-[2'-Hydroxyimino-3'-oxo-3'-(2"-{tert.-butoxycarbonylamino}-thiazol-4"-yl)-propionamido]-3-(acetoxymethyl)-ceph-3-em-4-carbonsäure mit $R_f$-Werten von 0,30 und 0,27 bei der Dünnschichtchromatographie [Silicagel F 254 : Eluiermittel : Gemisch von Chloroform und Methanol im Volumverhältnis von 7 : 3] erhalten.

Es wurde eine Lösung von 0,80 g der wie vorstehend beschrieben erhaltenen 7β-[2'-Hydroxyimino-3'-oxo-3'-(2"-{tert.-butoxycarbonylamino}-thiazol-4"-yl)-propionamido]-3-(acetoxymethyl)-ceph-3-em-4-carbonsäure, 3,2 $cm^3$ Anisol und 6,4 $cm^3$ Trifluoressigsäure 75 Minuten lang bei Raumtemperatur gerührt beziehungsweise geschüttelt, dann unter Vakuum eingedampft und in ein Gemisch von 50 $cm^3$ Diäthyläther und 50 $cm^3$ einer 3 %-igen Natriumbicarbonatlösung aufgenommen. Die Schichten wurden voneinander getrennt und zur wäßrigen Schicht wurden 50 $cm^3$ Tetrahydrofuran und Salzsäure bis zur Erreichung eines pH-Wertes von 3 zugegeben, worauf Natriumchlorid bis zur Sättigung zugesetzt wurde. Die Schichten wurden voneinander getrennt und die organische Schicht wurde mit einer gesättigten Natriumchloridlösung gewaschen, über wasserfreiem Magnesiumsulfat wasserfrei gemacht und unter Vakuum zur Trockne eingedampft. Der Rückstand wurde in Aceton aufgenommen, der feste Stoff wurde abfiltriert, die Lösung wurde unter Vakuum zur Trockne eingedampft und der so erhaltene Rückstand wurde in Diäthyläther verrieben und abfiltriert. So wurde 0,5 g (75,8 % der Theorie) 7β-[2'-Hydroxyimino-3'-oxo-3'-(2"-aminothiazol-4"-yl)-propionamido]-3-(acetoxymethyl)-ceph-3-em-4-carbonsäure mit einem Schmelzpunkt über 140 °C (unter Zersetzung) und $R_f$-Werten von 0,70 und 0,57 bei der Dünnschichtchromatographie [Silicagel F 254 ; Eluiermittel : Gemisch von Äthylacetat und Essigsäure im Volumverhältnis von 6 : 2] erhalten.

Das als Ausgangsverbindung verwendete 2,2-Dimethyl-5-[1'-hydroxy-1'-(2"-{tert.-butoxycarbonylamino}-thiazol-4"-yl)-methyliden]-4,6-dioxo-1,3-dioxan ist wie folgt beschrieben erhalten worden.

Es wurde eine Lösung von 21,4 g 2-(tert.-Butoxycarbonyl)-aminothiazol-4-ylcarbonsäure, 13,1 $cm^3$ Triäthylamin und 500 $cm^3$ wasserfreiem Tetrahydrofuran auf 0 °C gekühlt und mit 9,17 $cm^3$ Chlorameisensäureäthylester versetzt. Es wurde 30 Minuten lang bei 0 °C gerührt beziehungsweise geschüttelt, das Triäthylaminhydrochlorid wurde unter Vakuum abfiltriert und die erhaltene Lösung wurde zu einer Lösung von 12,7 g 2,2-Dimethyl-4,6-dioxo-1,3-dioxan, 9,6 g 50 %-igem Natriumhydrid in Öl und 400 $cm^3$ wasserfreiem Tetrahydrofuran zugegeben und 1 Stunde lang bei Raumtemperatur umgesetzt. Die Reaktionsmischung wurde noch 20 Stunden bei Raumtemperatur gerührt beziehungsweise geschüttelt und dann wurde das Lösungsmittel unter Vakuum abgedampft. Der Rückstand wurde in ein Gemisch von 400 $cm^3$ Äthylacetat und 100 $cm^3$ einer n Salzsäure aufgenommen, die organische Schicht wurde abgetrennt und über wasserfreiem Magnesiumsulfat wasserfrei gemacht, das Lösungsmittel wurde unter Vakuum abgedampft und der Rückstand wurde in Isopropyläther verrieben und unter Vakuum filtriert. So wurden 18 g (55,2 % der Theorie) 2,2-Dimethyl-5-[1'-hydroxy-1'-(2"-{tert.-butoxycarbonylamino}-thiazol-4"-yl)-methyliden]-4,6-dioxo-1,3-dioxan mit einem Schmelzpunkt von 240 bis 250° (unter Zersetzung) erhalten.

**Patentansprüche** (für die Vertragsstaaten : BE, CH, DE, FR, GB, IT, LI, LU, NL, SE)

1. 7β-(Ketooximinoacylamido)-ceph-3-em-4-carbonsäure-derivate der allgemeinen Formel

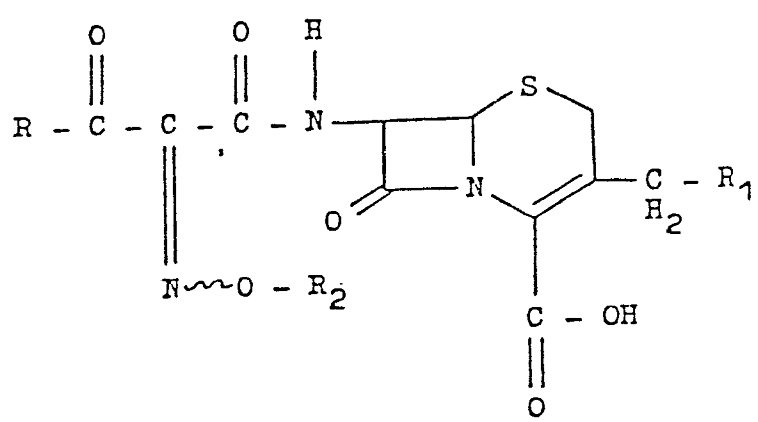

(I)

worin

R für einen Alkylrest mit 2 bis 4 Kohlenstoffatomen, einen Phenylrest oder einen, gegebenenfalls aminosubstituierten, als Heteroatom(e) Stickstoff, Sauerstoff und/oder Schwefel aufweisenden heterocyclischen Rest mit 5 Ringatomen steht,

$R_1$ einen Acetoxyrest, einen Carbamoyloxyrest oder einen Rest der allgemeinen Formel

—S—Het (II)

in welchletzterer

Het für einen, gegebenenfalls durch einen Alkyl- oder Sulfoalkylrest mit 1 bis 4 Kohlenstoffatom(en) oder eine Carboxylgruppe substituierten, heterocyclischen Rest mit 5 oder 6 Ringatomen und 1 oder mehr Stickstoffatom(en) und gegebenenfalls Schwefelatom(en) als Heteroatom(en) steht,

bedeutet,

$R_2$ Wasserstoff, einen Alkylrest mit 1 bis 4 Kohlenstoffatom(en) oder einen Acetylrest darstellt und die Wellenlinie das Vorliegen der syn- oder anti-Konfiguration oder von beiden derselben je nach der Konfiguration der Oximinogruppe bedeutet,

sowie ihre Alkalimetallsalze.

2. 7β-(Ketooximinoacylamido)-ceph-3-em-4-carbonsäurederivate nach Anspruch 1, dadurch gekennzeichnet, daß der Alkylrest, für den R stehen kann, ein solcher mit 2 oder 3 Kohlenstoffatomen ist.

3. 7β-(Ketooximinoacylamido)-ceph-3-em-4-carbonsäurederivate nach Anspruch 1, dadurch gekennzeichnet, daß der heterocyclische Rest, für den R stehen kann, ein Furylrest, ein Thienylrest oder ein, gegebenenfalls aminosubstituierter, Thiazolylrest ist.

4. 7β-(Ketooximinoacylamido)-ceph-3-em-4-carbonsäurederivate nach Anspruch 1 bis 3, dadurch gekennzeichnet, daß der heterocyclische Rest, für welchen Het steht, ein Tetrazolyl-, Thiazolyl-, Thiadiazolyl- oder Pyridazinylrest ist.

5. 7β-(Ketooximinoacylamido)-ceph-3-em-4-carbonsäurederivate nach Anspruch 1 bis 4, dadurch gekennzeichnet, daß der Alkyl- beziehungsweise Sulfoalkylrest, durch welchen der heterocyclische Rest, für den Het steht, substituiert sein kann, ein solcher mit 1 bis 3, insbesondere 1 oder 2 Kohlenstoffatom(en) ist.

6. 7β-(Ketooximinoacylamido)-ceph-3-em-4-carbonsäurederivate nach Anspruch 1 bis 5, dadurch gekennzeichnet, daß der Alkylrest, für den $R_2$ stehen kann, ein solcher mit 1 oder 2 Kohlenstoffatom(en) ist.

7. 7β-(2'-Hydroxyimino-3'-oxovaleramido)-3-(acetoxymethyl)-ceph-3-em-4-carbonsäure.

8. 7β-(2'-Hydroxyimino-3'-oxo-4'-methylvaleramido)-3-(acetoxymethyl)-ceph-3-em-4-carbonsäure.

9. 7β-(2'-Hydroxyimino-3'-oxovaleramido)-3-[(1"-methyl-1"H-tetrazol-5"-yl)-thiomethyl]-ceph-3-em-4-carbonsäure.

10. Verfahren zur Herstellung der Verbindungen nach Anspruch 1 bis 9, dadurch gekennzeichnet, daß man

a) 7β-Aminocephalosporansäure beziehungsweise Derivate derselben der allgemeinen Formel

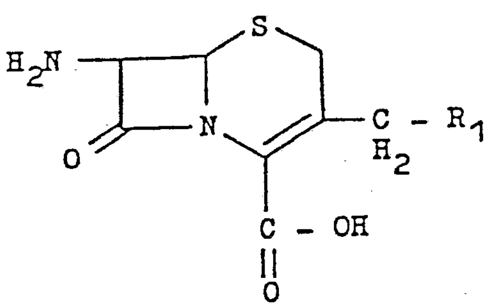

(III)

worin $R_1$ wie im Anspruch 1, 4 oder 5 festgelegt ist, in geschützter Form in aprotonischen Lösungsmitteln bei Temperaturen von 25 bis 100 °C mit 2,2-Dimethyl-4,6-dioxo-5-acyl-1,3-dioxanen der allgemeinen Formel

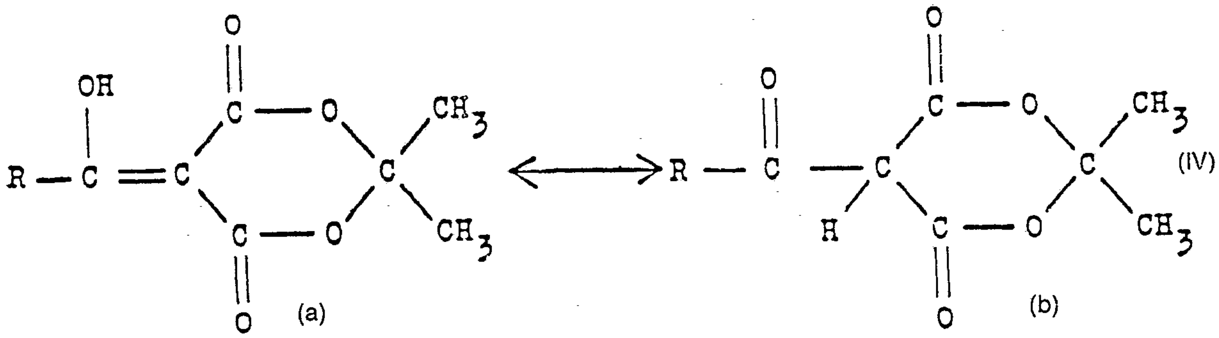

(IV)

worin R wie in den Ansprüchen 1 bis 3 festgelegt ist, zu 7β-(Ketoacylamido)-ceph-3-em-4-carbonsäurederivaten der allgemeinen Formel

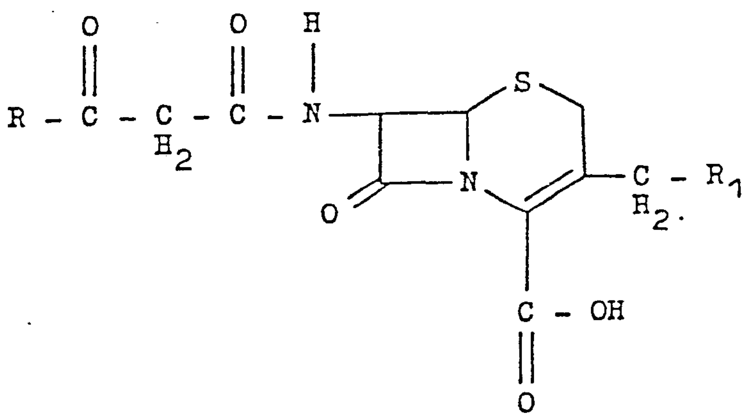

(V)

worin R und $R_1$ wie in den Ansprüchen 1 bis 5 festgelegt sind, umsetzt und diese, gegebenenfalls in geschützter Form, in an sich bekannter Weise mit Lösungen von Alkalinitriten in Gegenwart von Essigsäure oder Ameisensäure zu 7β-(Ketooximinoacylamido)-ceph-3-em-4-carbonsäurederivaten der allgemeinen Formel I, bei welchen $R_2$ Wasserstoff bedeutet, nitrosiert und

α) gegebenenfalls diese in an sich bekannter Weise, alkyliert oder acetyliert und/oder

β) gegebenenfalls, soweit 7β-(Ketooximinoacylamido)-ceph-3-em-4-carbonsäurederivate der allgemeinen Formel I, bei welchen $R_1$ einen Acetoxyrest bedeutet, erhalten wurden, diese in an sich bekannter Weise in die entsprechenden 7β-(Ketooximinoacylamido)-ceph-3-em-4-carbonsäurederivate der allgemeinen Formel I, bei welchen $R_1$ einen Carbamoyloxyrest oder einen Rest der allgemeinen Formel II bedeutet, überführt oder

b) in an sich bekannter Weise 3-Oxo-2-alkoxyiminoalkansäuren beziehungsweise 3-Oxo-2-acetoxyiminoalkansäuren der allgemeinen Formel

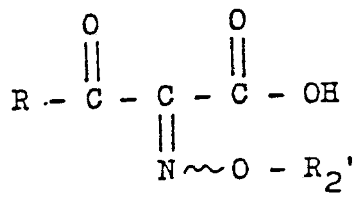

(VI)

worin $R_2'$ für einen Alkylrest mit 1 bis 4 Kohlenstoffatom(en) oder einen Acetylrest steht und R wie in den Ansprüchen 1 bis 3 festgelegt ist, oder reaktionsfähige funktionelle Derivate derselben mit 7β-Aminocephalosporansäure beziehungsweise Derivaten derselben der allgemeinen Formel

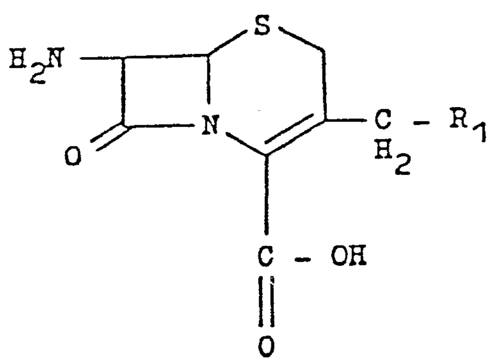

(III)

worin $R_1$ wie im Anspruch 1, 4 oder 5 festgelegt ist, in geschützter Form zu 7β-(Ketooximinoacylamido)-ceph-3-em-4-carbonsäurederivaten der allgemeinen Formel I, bei welchen $R_2$ einen Alkylrest mit 1 bis 4 Kohlenstoffatom(en) oder einen Acetylrest bedeutet, umsetzt,
worauf man gegebenenfalls vorliegende Schutzgruppen in an sich bekannter Weise entfernt und/oder gegebenenfalls die erhaltenen 7β-(Ketooximinoacylamido)-ceph-3-em-4-carbonsäurederivate der allgemeinen Formel I in an sich bekannter Weise in Alkalisalze überführt.

11. Arzneimittel, gekennzeichnet durch einen Gehalt an 1 oder mehr Verbindungen nach Anspruch 1 bis 9 als Wirkstoff beziehungsweise Wirkstoffen, zweckmäßig zusammen mit 1 oder mehr üblichen pharmazeutischen Konfektionierungsmittel(n).

**Patentansprüche** (für den Vertragsstaat AT)

1. Verfahren zur Herstellung von 7β-(Ketooximinoacylamido)-ceph-3-em-4-carbonsäurederivaten der allgemeinen Formel

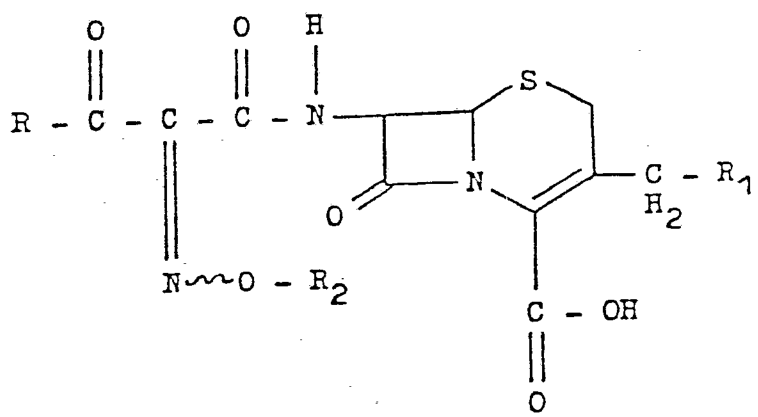

(I)

worin

R für einen Alkylrest mit 2 bis 4 Kohlenstoffatomen, einen Phenylrest oder einen, gegebenenfalls aminosubstituierten, als Heteroatom(e) Stickstoff, Sauerstoff und/oder Schwefel aufweisenden heterocyclischen Rest mit 5 Ringatomen steht,

$R_1$ einen Acetoxyrest, einen Carbamoyloxyrest oder einen Rest der allgemeinen Formel

—S—Het (II)

in welchletzterer

Het für einen, gegebenenfalls durch einen Alkyl- oder Sulfoalkylrest mit 1 bis 4 Kohlenstoffatom(en) oder eine Carboxylgruppe substituierten, heterocyclischen Rest mit 5 oder 6 Ringatomen und 1 oder mehr Stickstoffatom(en) und gegebenenfalls Schwefelatom(en) als Heteroatom(en) steht,

bedeutet,

$R_2$ Wasserstoff, einen Alkylrest mit 1 bis 4 Kohlenstoffatom(en) oder einen Acetylrest darstellt und die Wellenlinie das Vorliegen der syn- oder anti-Konfiguration oder von beiden derselben je nach der Konfiguration der Oximinogruppe bedeutet,

sowie ihren Alkalimetallsalzen, dadurch gekennzeichnet, daß man

a) 7β-Aminocephalosporansäure beziehungsweise Derivate derselben der allgemeinen Formel

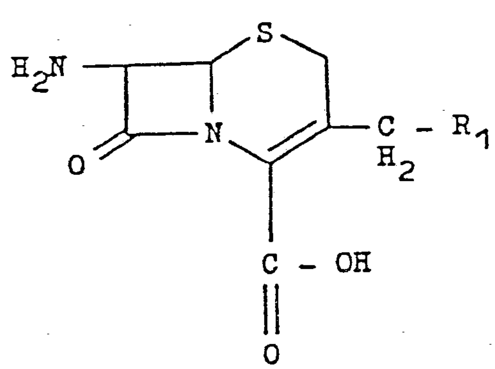

(III)

worin $R_1$ wie oben festgelegt ist, in geschützter Form in aprotonischen Lösungsmitteln bei Temperaturen von 25 bis 100 °C mit 2,2-Dimethyl-4,6-dioxo-5-acyl-1,3-dioxanen der allgemeinen Formel

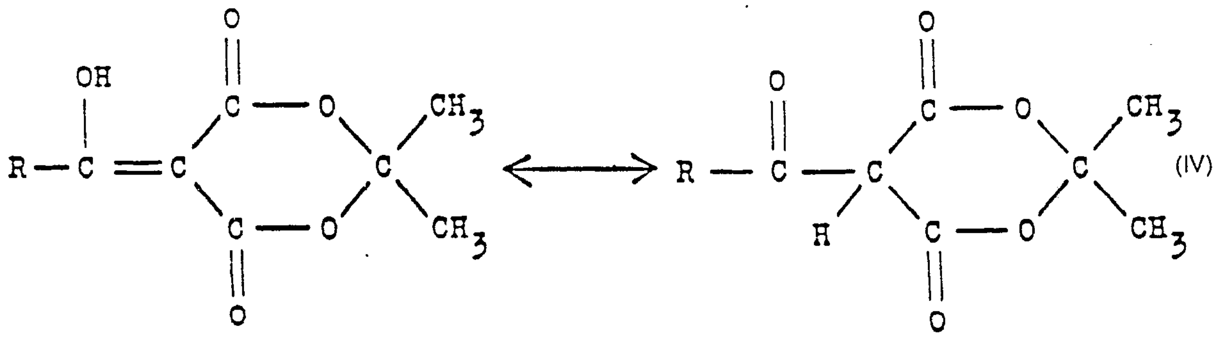

(IV)

worin R wie oben festgelegt ist, zu 7β-(Ketoacylamido)-ceph-3-em-4-carbonsäurederivaten der allgemeinen Formel

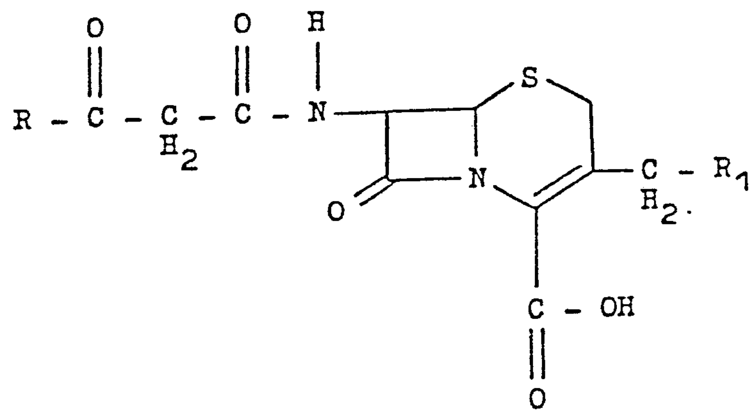

(V)

worin R und $R_1$ wie oben festgelegt sind, umsetzt und diese, gegebenenfalls in geschützter Form, in an sich bekannter Weise mit Lösungen von Alkalinitriten in Gegenwart von Essigsäure oder Ameisensäure zu 7β-(Ketooximinoacylamido)-ceph-3-em-4-carbonsäurederivaten der allgemeinen Formel I, bei welchen $R_2$ Wasserstoff bedeutet, nitrosiert und

α) gegebenenfalls diese in an sich bekannter Weise, alkyliert oder acetyliert und/oder

β) gegebenenfalls, soweit 7β-(Ketooximinoacylamido)-ceph-3-em-4-carbonsäurederivate der allgemeinen Formel I, bei welchen $R_1$ einen Acetoxyrest bedeutet, erhalten wurden, diese in an sich bekannter Weise in die entsprechenden 7β-(Ketooximinoacylamido)-ceph-3-em-4-carbonsäurederivate der allgemeinen Formel I, bei welchen $R_1$ einen Carbamoyloxyrest oder einen Rest der allgemeinen Formel II bedeutet, überführt oder

b) in an sich bekannter Weise 3-Oxo-2-alkoxyiminoalkansäuren beziehungsweise 3-Oxo-2-acetoxyiminoalkansäuren der allgemeinen Formel

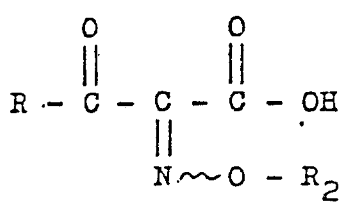

(VI)

worin $R_2'$ für einen Alkylrest mit 1 bis 4 Kohlenstoffatom(en) oder einen Acetylrest steht und R wie oben festgelegt ist, oder reaktionsfähige funktionelle Derivate derselben mit 7β-Aminocephalosporansäure beziehungsweise Derivaten derselben der allgemeinen Formel

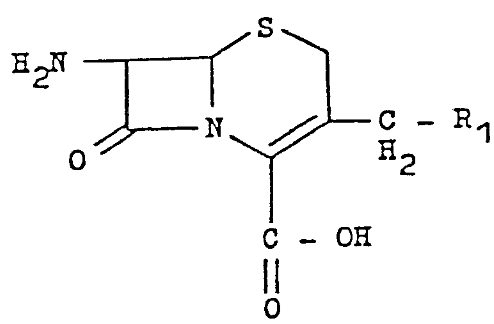

(III)

worin $R_1$ wie oben festgelegt ist, in geschützter Form zu 7β-(Ketooximinoacylamido)-ceph-3-em-4-carbonsäurederivaten der allgemeinen Formel I, bei welchen $R_2$ einen Alkylrest mit 1 bis 4 Kohlenstoffatom(en) oder einen Acetylrest bedeutet, umsetzt,
worauf man gegebenenfalls vorliegende Schutzgruppen in an sich bekannter Weise entfernt und/oder gegebenenfalls die erhaltenen 7β-(Ketooximinoacylamido)-ceph-3-em-4-carbonsäurederivate der allgemeinen Formel I in an sich bekannter Weise in Alkalisalze überführt.

2. Verfahren nach Anspruch 1, dadurch gekennzeichnet, daß man als 7β-(Ketooximinoacylamido)-ceph-3-em-4-carbonsäurederivate solche, bei denen der Alkylrest, für den R stehen kann, ein solcher mit 2 oder 3 Kohlenstoffatomen ist, herstellt.

3. Verfahren nach Anspruch 1, dadurch gekennzeichnet, daß man als 7β-(Ketooximinoacylamido)-ceph-3-em-4-carbonsäurederivate solche, bei denen der heterocyclische Rest, für den R stehen kann, ein Furylrest, ein Thienylrest oder ein, gegebenenfalls aminosubstituierter, Thiazolylrest ist, herstellt.

4. Verfahren nach Anspruch 1 bis 3, dadurch gekennzeichnet, daß man als 7β-(Ketooximinoacylamido)-ceph-3-em-4-carbonsäurederivate solche, bei denen der heterocyclische Rest, für welchen Het steht, ein Tetrazolyl-, Thiazolyl-, Thiadiazolyl- oder Pyridazinylrest ist, herstellt.

5. Verfahren nach Anspruch 1 bis 4, dadurch gekennzeichnet, daß man als 7β-(Ketooximinoacylamido)-ceph-3-em-4-carbonsäurederivate solche, bei denen der Alkyl- beziehungsweise Sulfoalkylrest, durch welchen der heterocyclische Rest, für den Het steht, substituiert sein kann, ein solcher mit 1 bis 3, insbesondere 1 oder 2 Kohlenstoffatom(en) ist, herstellt.

6. Verfahren nach Anspruch 1 bis 5, dadurch gekennzeichnet, daß man als 7β-(Ketooximinoacylamido)-ceph-3-em-4-carbonsäurederivate solche, bei denen der Alkylrest, für den $R_2$ stehen kann, ein solcher mit 1 oder 2 Kohlenstoffatom(en) ist, herstellt.

7. Verfahren nach Anspruch 1 oder 2, dadurch gekennzeichnet, daß man 7β-(2'-Hydroxyimino-3'-oxovaleramido)-3-(acetoxymethyl)-ceph-3-em-4-carbonsäure herstellt.

8. Verfahren nach Anspruch 1 oder 2, dadurch gekennzeichnet, daß man 7β-(2'-Hydroxyimino-3'-oxo-4'-methylvaleramido)-3-(acetoxymethyl)-ceph-3-em-4-carbonsäure herstellt.

9. Verfahren nach Anspruch 1, 4 oder 5, dadurch gekennzeichnet, daß man 7β-(2'-Hydroxyimino-3'-oxovaleramido)-3-[(1''-methyl-1''H-tetrazol-5''-yl)-thiomethyl]-ceph-3-em-4-carbonsäure herstellt.

**Claims** (for the Contracting States : BE, CH, DE, FR, GB, IT, LI, LU, NL, SE)

1. 7β-(Ketoximinoacylamido)-ceph-3-em-4-carboxylic acid derivatives having the general formula

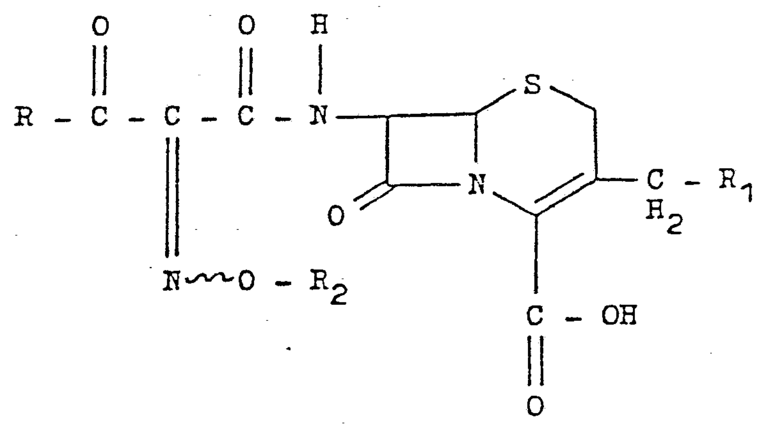

(I)

wherein

R represents an alkyl radical having from 2 to 4 carbon atoms, a phenyl radical or a heterocyclic radical having 5 ring atoms and nitrogen, oxygen and/or sulphur as [a] hetero atom(s), optionally aminosubstituted,

$R_1$ means an acetoxy radical, a carbamoyloxy radical or a radical having the general formula

—S—Het (II)

in which latter

Het represents a heterocyclic radical having 5 or 6 ring atoms and 1 or more nitrogen atom(s) and optionally sulphur atom(s) as [an] hetero atom(s), optionally substituted by an alkyl or sulphoalkyl radical having from 1 to 4 carbon atom(s) or a carboxylic group,

$R_2$ represents hydrogen, an alkyl radical having from 1 to 4 carbon atom(s) or an acetyl radical and the wave line means the presence of the syn- or anti-configuration or of both of them according to the configuration of the oximino group

as well as their alkali metal salts.

2. 7β-(Ketoximinoacylamido)-ceph-3-em-4-carboxylic acid derivatives according to claim 1, characterized in that the alkyl radical which may be represented by R is such one having 2 or 3 carbon atoms.

3. 7β-(Ketoximinoacylamido)-ceph-3-em-4-carboxylic acid derivatives according to claim 1, characterized in that the heterocyclic radical which may be represented by R is a furyl radical, a thienyl radical or a thiazolyl radical, optionally aminosubstituted.

4. 7β-(Ketoximinoacylamido)-ceph-3-em-4-carboxylic acid derivatives according to claims 1 to 3, characterized in that the heterocyclic radical which may be represented by Het is a tetrazolyl, thiazolyl, thiadiazolyl or pyridazinyl radical.

5. 7β-(Ketoximinoacylamido)-ceph-3-em-4-carboxylic acid derivatives according to claims 1 to 4, characterized in that the alkyl or sulphoalkyl radical, respectively, by which the heterocyclic radical which may be represented by Het may be substituted is such one having from 1 to 3, particularly 1 or 2 carbon atom(s).

6. 7β-(Ketoximinoacylamido)-ceph-3-em-4-carboxylic acid derivatives according to claims 1 to 5, characterized in that the alkyl radical which may be represented by $R_2$ is such one having 1 or 2 carbon atom(s).

7. 7β-(2'-Hydroxyimino-3'-oxovaleramido)-3-(acetoxymethyl)-ceph-3-em-4-carboxylic acid.

8. 7β-(2'-Hydroxyimino-3'-oxo-4'-methylvaleramido)-3-(acetoxymethyl)-ceph-3-em-4-carboxylic acid.

9. 7β-(2'-Hydroxyimino-3'-oxovaleramido)-3-[(1"-methyl-1"H-tetrazol-5"-yl)-thiomethyl]-ceph-3-em-4-carboxylic acid.

10. A process for preparing the compounds according to claims 1 to 9, characterized in that

a) one reacts 7β-aminocephalosporanic acid or derivatives of it, respectively, having the general formula

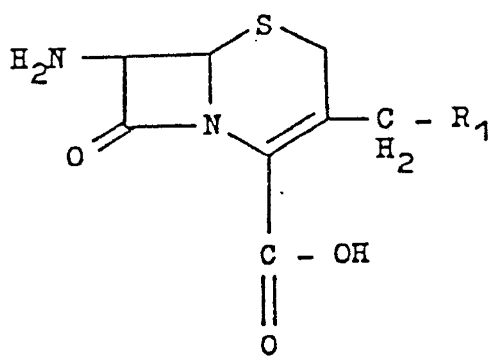

(III)

wherein $R_1$ is as defined in claim 1, 4 or 5 in protected form in aprotic solvents at temperatures of from 25 to 100 °C with 2,2-dimethyl-4,6-dioxo-5-acyl-1,3-dioxanes having the general formula

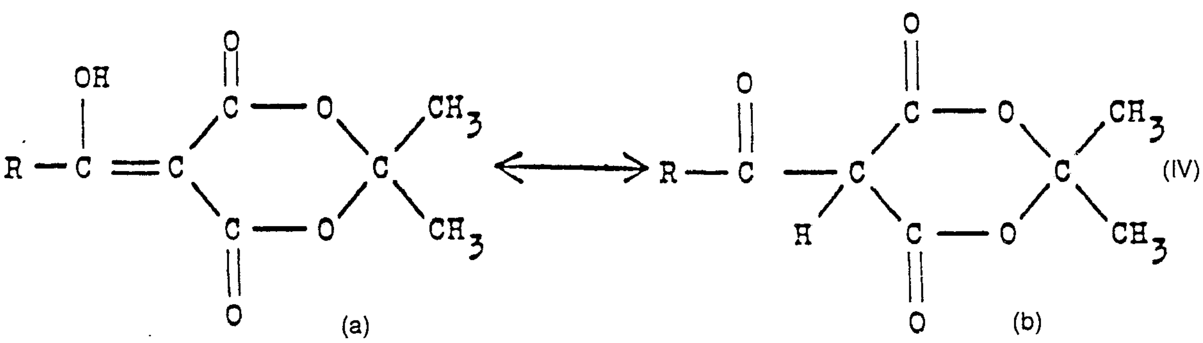

(a) (b) (IV)

wherein R is as defined in claims 1 to 3 to yield 7β-(ketoacylamido)-ceph-3-em-4-carboxylic acid derivatives having the general formula

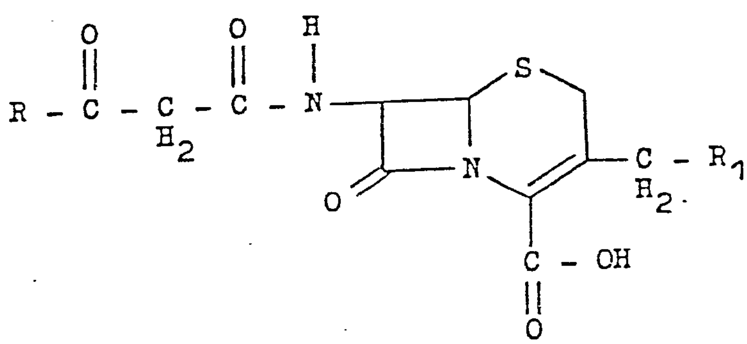

(V)

wherein R and $R_1$ are as defined in claims 1 to 5 and one nitrosates these, optionally in protected form, in a manner known per se with solutions of alkali nitrites in the presence of acetic acid or formic acid to yield 7β-(ketoximinoacylamido)-ceph-3-em-4-carboxylic acid derivatives having the general formula I in which $R_2$ means hydrogen and

α) optionally one alkylates or acetylates these in a manner known per se and/or

β) optionally one converts these insofar as 7β-(ketoximinoacylamido)-ceph-3-em-4-carboxylic acid derivatives having the general formula I in which $R_1$ means an acetoxy radical have been obtained in a manner known per se into the corresponding 7β-(ketoximinoacylamido)-ceph-3-em-4-carboxylic acid derivatives having the general formula I in which $R_1$ means a carbamoyloxy radical or a radical having the general formula II or

b) one reacts in a manner known per se 3-oxo-2-alkoxyiminoalkanoic acids or 3-oxo-2-acetoxyiminoalkanoic acids, respectively, having the general formula

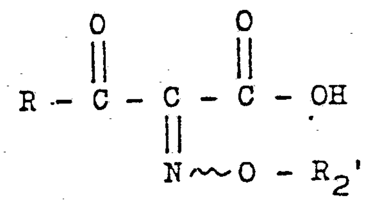

(VI)

wherein $R_2'$ represents an alkyl radical having from 1 to 4 carbon atom(s) or an acetyl radical and R is as defined in claims 1 to 3 or reactive functional derivatives of them with 7β-aminocephalosporanic acid or derivatives of it, respectively, having the general formula

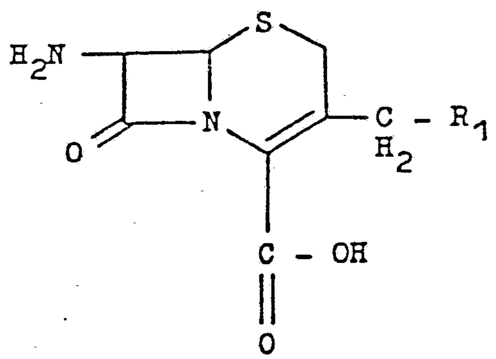

(III)

wherein $R_1$ is as defined in claim 1, 4 or 5 in protected form to yield 7β-(ketoximinoacylamido)-ceph-3-em-4-carboxylic acid derivatives having the general formula I in which $R_2$ means an alkyl radical having from 1 to 4 carbon atom(s) or an acetyl radical,
whereupon one eliminates protective groups optionally present in a manner known per se and/or optionally one converts the obtained 7β-(ketoximinoacylamido)-ceph-3-em-4-carboxylic acid derivatives having the general formula I in a manner known per se into alkali salts.

11. Medicaments, characterized by a content of 1 or more compounds according to claims 1 to 9 as an active principle or active principles, respectively, usefully together with 1 or more usual pharmaceutical processing agent(s).

**Claims** (for the Contracting State AT)

1. A process for preparing 7β-(ketoximinoacylamido)-ceph-3-em-4-carboxylic acid derivatives having the general formula

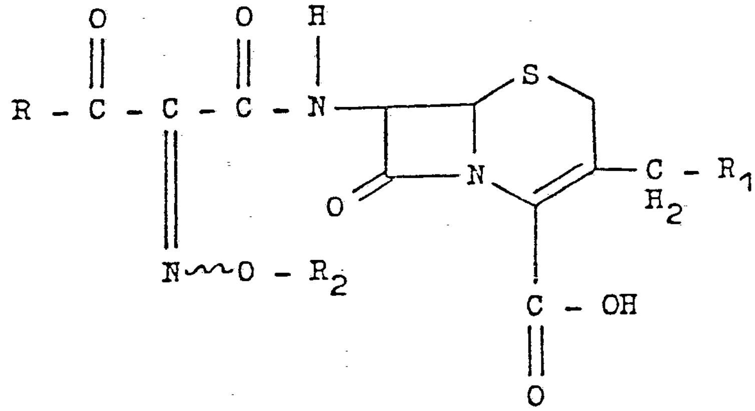

(I)

wherein

R represents an alkyl radical having from 2 to 4 carbon atoms, a phenyl radical or a heterocyclic radical having 5 ring atoms and nitrogen, oxygen and/or sulphur as [a] hetero atom(s), optionally aminosubstituted,

$R_1$ means an acetoxy radical, a carbamoyloxy radical or a radical having the general formula

—S—Het (II)

in which latter

Het represents a heterocyclic radical having 5 or 6 ring atoms and 1 or more nitrogen atom(s) and optionally sulphur atom(s) as [an] hetero atom(s), optionally substituted by an alkyl or sulphoalkyl radical having from 1 to 4 carbon atom(s) or a carboxylic group,

$R_2$ represents hydrogen, an alkyl radical having from 1 to 4 carbon atom(s) or an acetyl radical and

the wave line means the presence of the syn- or anti-configuration or of both of them according to the configuration of the oximino group

as well as their alkali metal salts, characterized in that

a) one reacts 7β-aminocephalosporanic acid or derivatives of it, respectively, having the general formula

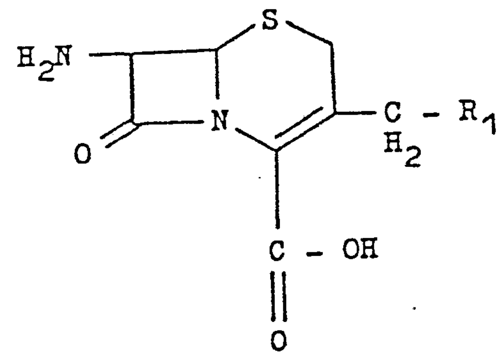

(III)

wherein $R_1$ is as defined above in protected form in aprotic solvents at temperatures of from 25 to 100 °C with 2,2-dimethyl-4,6-dioxo-5-acyl-1,3-dioxanes having the general formula

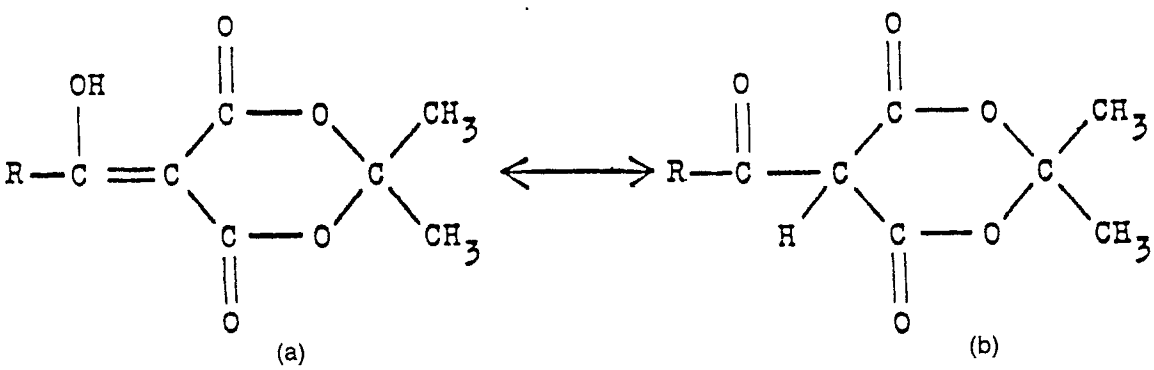

(a) (b) (IV)

wherein R is as defined above to yield 7β-(ketoacylamido)-ceph-3-em-4-carboxylic acid derivatives having the general formula

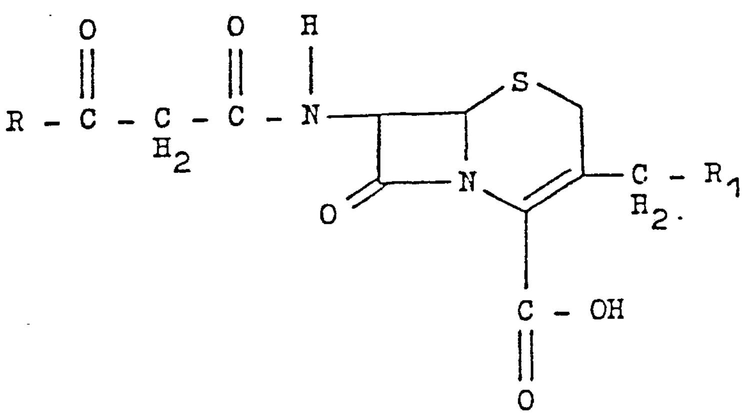

(V)

wherein R and $R_1$ are as defined above and one nitrosates these, optionally in protected form, in a manner known per se with solutions of alkali nitrites in the presence of acetic acid or formic acid to yield 7β-(ketoximinoacylamino)-ceph-3-em-4-carboxylic acid derivatives having the general formula I in which $R_2$ means hydrogen and

α) optionally one alkylates or acetylates these in a manner known per se and/or

β) optionally one converts these insofar as 7β-(ketoxyiminoacylamido)-ceph-3-em-4-carboxylic acid derivatives having the general formula I in which $R_1$ means an acetoxy radical have been obtained in a manner known per se into the corresponding 7β-(ketoxyiminoacylamino)-ceph-3-em-4-carboxylic acid derivatives having the general formula I in which $R_1$ means a carbamoyloxy radical or a radical having the general formula II or

b) one reacts in a manner known per se 3-oxo-2-alkoxyiminoalkanoic acids or 3-oxo-2-acetoxyiminoalkanoic acids, respectively, having the general formula

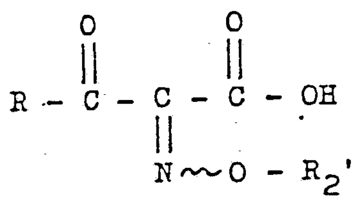

(VI)

wherein $R_2'$ represents an alkyl radical having from 1 to 4 carbon atom(s) or an acetyl radical and R is as defined above or reactive functional derivatives of them with 7β-aminocephalosporanic acid or derivatives of it, respectively, having the general formula

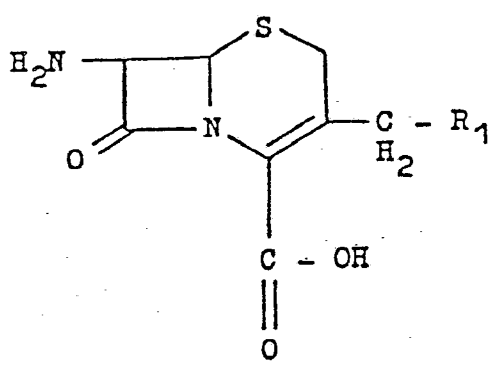

(III)

wherein $R_1$ is as defined above in protected form to yield 7β-(ketoximinoacylamido)-ceph-3-em-4-carboxylic acid derivatives having the general formula I in which $R_2$ means an alkyl radical having from 1 to 4 carbon atom(s) or an acetyl radical,
whereupon one eliminates protective groups optionally present in a manner known per se and/or optionally one converts the obtained 7β-(ketoximinoacylamido)-ceph-3-em-4-carboxylic acid derivatives having the general formula I in a manner known per se into alkali salts.

2. A process according to claim 1, characterized in that one prepares as 7β-(ketoximinoacylamido)-ceph-3-em-4-carboxylic acid derivatives such ones in which the alkyl radical which may be represented by R is such one having 2 or 3 carbon atoms.

3. A process according to claim 1, characterized in that one prepares as 7β-(ketoximinoacylamido)-ceph-3-em-4-carboxylic acid derivatives such ones in which the heterocyclic radical which may be represented by R is a furyl radical, a thienyl radical or a thiazolyl radical, optionally aminosubstituted.

4. A process according to claims 1 to 3, characterized in that one prepares as 7β-(ketoximinoacylamido)-ceph-3-em-4-carboxylic acid derivatives such ones in which the heterocyclic radical which may be represented by Het is a tetrazolyl, thiazolyl, thiadiazolyl or pyridazinyl radical.

5. A process according to claims 1 to 4, characterized in that one prepares as 7β-(ketoximinoacylamido)-ceph-3-em-4-carboxylic acid derivatives such ones in which the alkyl or sulphoalkyl radical, respectively, by which the heterocyclic radical which may be represented by Het may be substituted is such one having from 1 to 3, particularly 1 or 2 carbon atom(s).

6. A process according to claims 1 to 5, characterized in that one prepares as 7β-(ketoximinoacylamido)-ceph-3-em-4-carboxylic acid derivatives such ones in which the alkyl radical which may be represented by $R_2$ is such one having 1 or 2 carbon atom(s).

7. A process according to claim 1 or 2, characterized in that one prepares 7β-(2'-hydroxyimino-3'-oxovaleramido)-3-(acetoxymethyl)-ceph-3-em-4-carboxylic acid.

8. A process according to claim 1 or 2, characterized in that one prepares 7β-(2'-hydroxyimino-3'-oxo-4'-methylvaleramido)-3-(acetoxymethyl)-ceph-3-em-4-carboxylic acid.

9. A process according to claim 1, 4 or 5, characterized in that one prepares 7β-(2'-hydroxyimino-3'-oxovaleramido)-3-[(1"-methyl-1"H-tetrazol-5"-yl)-thiomethyl]-ceph-3-em-4-carboxylic acid.

**Revendications** (pour les Etats contractants : BE, CH, DE, FR, GB, IT, LI, LU, NL, SE)

1. Dérivés d'acides 7β-(cétoximinoacylamido)-céph-3-em-4-carboxyliques de formule générale

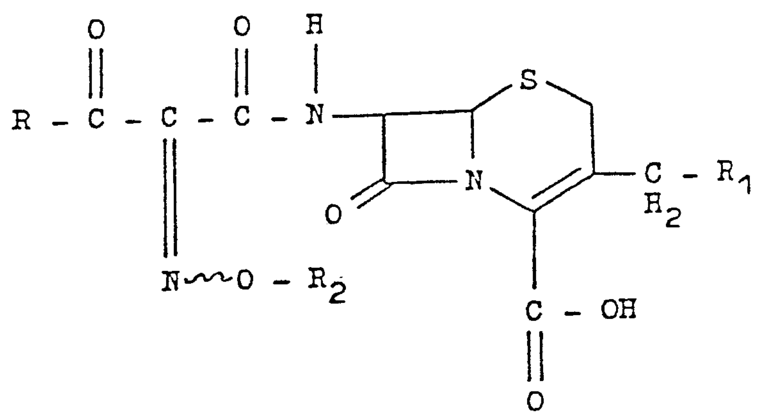

(I)

dans laquelle

R représente un reste alkyle avec 2 à 4 atomes de carbone, un reste phényle ou un reste hétérocyclique avec 5 atomes dans le noyau, éventuellement aminosubstitué, contenant de l'azote, de l'oxygène et/ou du soufre en tant qu'hétéroatome(s),

$R_1$ représente un reste acétoxy, un reste carbamoyloxy ou un reste de formule générale

—S—Het (II)

dans laquelle

Het désigne un reste hétérocyclique avec 5 ou 6 atomes dans le noyau et 1 ou plusieurs atomes d'azote et éventuellement un ou plusieurs atomes de soufre en tant qu'hétéroatomes, substitué par un reste alkyle ou sulfoalkyle possédant 1 à 4 atomes de carbone ou par un groupe carboxyle,

$R_2$ représente de l'hydrogène, un reste alkyle avec 1 à 4 atomes de carbone ou un reste acétyle et la ligne ondulée indique la présence de la configuration syn ou anti ou des deux selon la configuration du groupe oximino, ainsi que leurs sels de métaux alcalins.

2. Dérivés d'acides 7β-(cétoximinoacylamido)-céph-3-em-4-carboxyliques selon la revendication 1, caractérisés en ce que le reste alkyle qui peut être représenté par R est un reste avec 2 ou 3 atomes de carbone.

3. Dérivés d'acides 7β-(cétoximinoacylamido)-céph-3-em-4-carboxyliques selon la revendication 1, caractérisés en ce que le reste hétérocyclique, qui peut être représenté par R, est un reste furyle, un reste thiényle ou un reste thiazolyle, éventuellement aminosubstitué.

4. Dérivés d'acides 7β-(cétoximinoacylamido)-céph-3-em-4-carboxyliques selon les revendications 1 à 3, caractérisés en ce que le reste hétérocyclique représenté par Het est un reste tétrazolyle, thiazolyle, thiadiazolyle ou pyridazinyle.

5. Dérivés d'acides 7β-(cétoximinoacylamido)-céph-3-em-4-carboxyliques selon les revendications 1 à 4, caractérisés en ce que le reste alkyle, respectivement le reste sulfoalkyle par lequel peut être substitué le reste hétérocyclique représenté par Het, est un reste avec 1 à 3, en particulier avec 1 ou 2 atomes de carbone.

6. Dérivés d'acides 7β-(cétoximinoacylamido)-céph-3-em-4-carboxyliques selon les revendications 1 à 5, caractérisés en ce que le reste alkyle qui peut être représenté par $R_2$ est un reste avec 1 ou 2 atomes de carbone.

7. Acide 7β-(2'-hydroxyimino-3'-oxovaléramido)-3-(acétoxyméthyl)-céph-3-em-4-carboxylique.

8. Acide 7β-(2'-hydroxyimino-3'-oxo-4'-méthylvaléramido)-3-(acétoxyméthyl)-céph-3-em-4-carboxylique.

9. Acide 7β-(2'-hydroxyimino-3'-oxovaléramido)-3-[(1"-méthyl-1"H-tétrazol-5"-yl)-thiométhyl]-céph-3-em-4-carboxylique.

10. Procédé de préparation des composés selon les revendications 1 à 9, caractérisé en ce que

a) on fait réagir des acides 7β-aminocéphalosporaniques, respectivement des dérivés de ceux-ci répondant à la formule générale

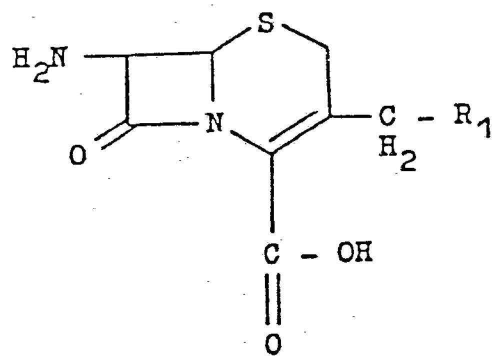

(III)

où $R_1$ est tel que défini dans la revendication 1, 4 ou 5, sous forme protégée dans des solvants aprotiques à des températures de 25 à 100 °C, avec des 2,2-diméthyl-4,6-dioxo-5-acyl-1,3-dioxannes de formule générale

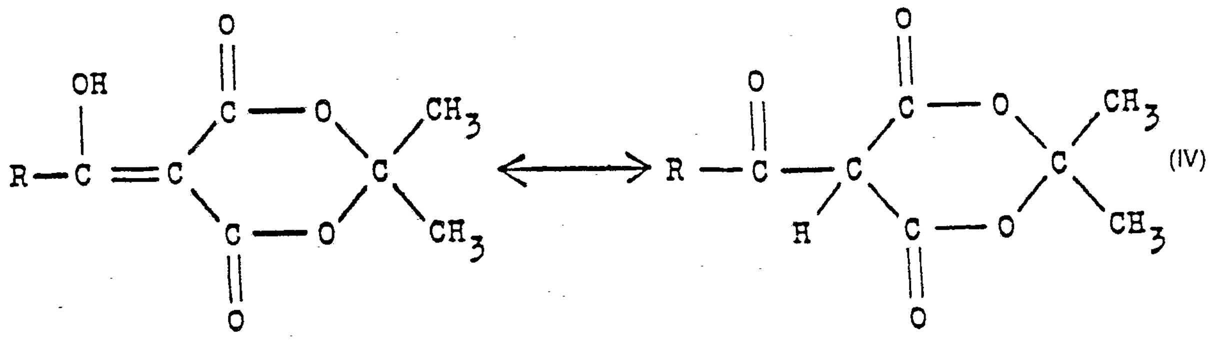

(a) (b)

(IV)

dans laquelle R est tel que défini dans les revendications 1 à 3, pour obtenir des dérivés d'acides 7β-(cétoacylamido)-céph-3-em-4-carboxyliques de formule générale

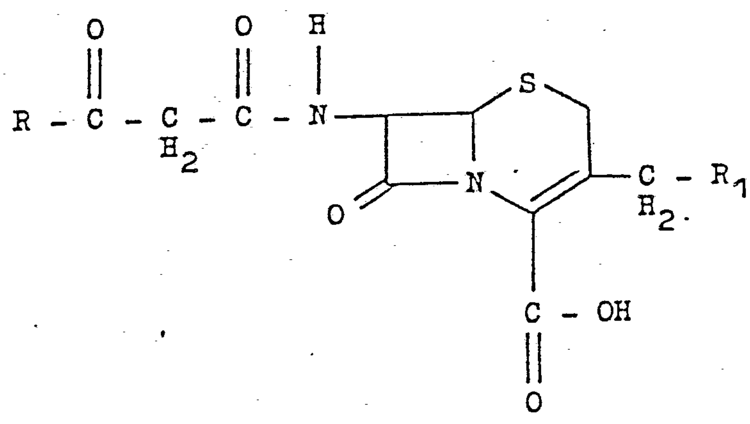

(V)

dans laquelle R et $R_1$ sont tels que définis dans les revendications 1 à 5, on nitrose ceux-ci de façon connue en soi, éventuellement sous forme protégée, avec des solutions de nitrites alcalins en présence d'acide acétique ou d'acide formique en vue d'obtenir des dérivés d'acides 7β-(cétoximinoacylamido)-céph-3-em-4-carboxyliques de formule I où $R_2$ représente de l'hydrogène, et

α) on alkyle ou acétyle ceux-ci éventuellement de façon connue en soi et/ou,

β) dans la mesure où ont été obtenus des dérivés d'acides 7β-(cétoximinoacylamido)-céph-3-em-4-carboxyliques de formule générale I où $R_1$ représente un reste acétoxy, on transforme éventuellement ceux-ci de façon connue en soi en les dérivés d'acides 7β-(cétoximinoacylamido)-céph-3-em-4-carboxyliques correspondants de formule générale I où $R_1$ représente un reste carbamoyloxy ou un reste de formule générale II, ou

b) on fait réagir de façon connue en soi des acides 3-oxo-alkoxyiminoalcaniques, respectivement des acides 3-oxo-acétoxyiminoalcaniques de formule générale

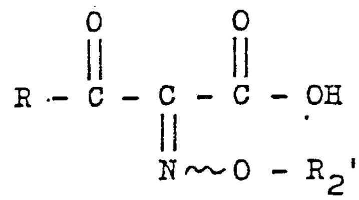

(VI)

dans laquelle $R_2'$ représente un reste alkyle avec 1 à 4 atomes de carbone ou un reste acétyle et R est tel que défini dans les revendications 1 à 3 ou leurs dérivés fonctionnels réactifs, avec des acides 7β-aminocéphalosporanniques ou leurs dérivés de formule générale

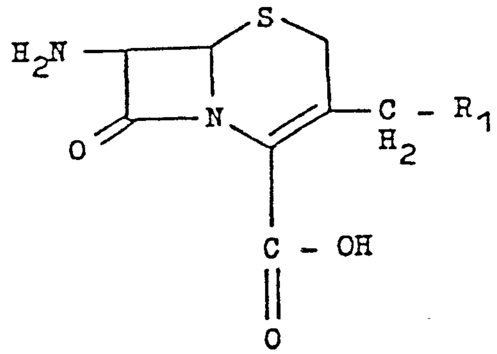

(III)

dans laquelle $R_1$ est tel que défini dans la revendication 1, 4 ou 5, sous forme protégée en vue d'obtenir des dérivés d'acides 7β-(cétoximinoacylamido)-céph-3-em-4-carboxyliques de formule générale I dans laquelle $R_2$ désigne un reste alkyle avec 1 à 4 atomes de carbone ou un reste acétyle, après quoi on élimine de façon connue en soi les groupes protecteurs éventuellement présents et/ou transforme éventuellement de façon connue en soi les dérivés d'acides 7β-(cétoximinoacylamido)-céph-3-em-4-carboxyliques obtenus de formule générale I, en des sels alcalins.

11. Produits pharmaceutiques, caractérisés en ce qu'ils contiennent 1 ou plusieurs composés selon les revendications 1 à 9 en tant que principe actif ou principes actifs, conjointement avec 1 ou plusieurs véhicules pharmaceutiques classiques.

**Revendications** (pour l'Etat contractant AT)

1. Procédé de préparation de dérivés d'acides 7β-(cétoximinoacylamido)-céph-3-em-4-carboxyliques de formule générale

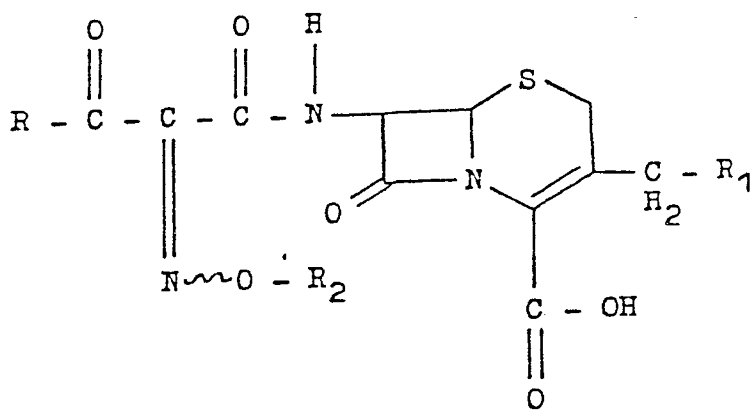

(I)

dans laquelle

R représente un reste alkyle avec 2 à 4 atomes de carbone, un reste phényle ou un reste hétérocyclique avec 5 atomes dans le noyau, éventuellement aminosubstitué, contenant de l'azote, de l'oxygène et/ou du soufre en tant qu'hétéroatome(s),

$R_1$ représente un reste acétoxy, un reste carbamoyloxy ou un reste de formule générale

—S—Het (II)

dans laquelle

Het désigne un reste hétérocyclique avec 5 ou 6 atomes dans le noyau et 1 ou plusieurs atomes

d'azote et éventuellement un ou plusieurs atomes de soufre en tant qu'hétéroatomes, substitué par un reste alkyle ou sulfoalkyle possédant 1 à 4 atomes de carbone ou par un groupe carboxyle,

$R_2$ représente de l'hydrogène, un reste alkyle avec 1 à 4 atomes de carbone ou un reste acétyle et la ligne ondulée indique la présence de la configuration syn ou anti ou des deux selon la configuration du groupe oximino, ainsi que leurs sels de métaux alcalins, caractérisé en ce que

a) on fait réagir des acides 7β-aminocéphalosporanniques, respectivement des dérivés de ceux-ci répondant à la formule générale

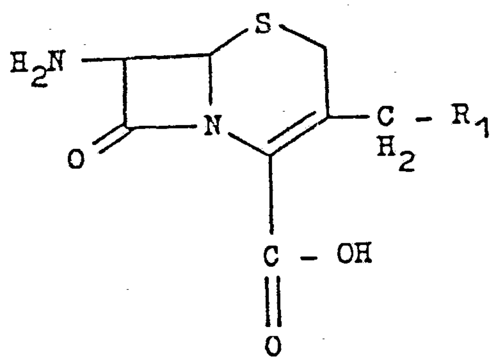

(III)

où $R_1$ est tel que défini plus haut sous forme protégée dans des solvants aprotiques à des températures de 25 à 100 °C, avec des 2,2-diméthyl-4,6-dioxo-5-acyl-1,3-dioxannes de formule générale

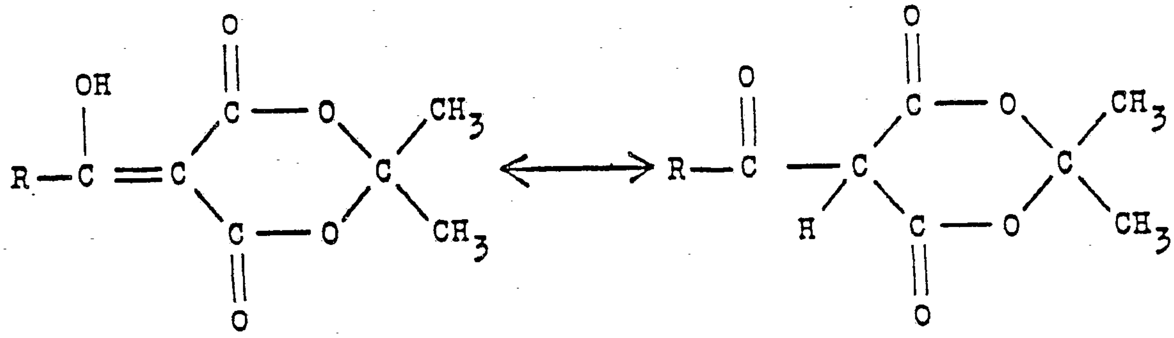

(IV)

(a) (b)

dans laquelle R est tel que défini plus haut, pour obtenir des dérivés d'acides 7β-(cétoacylamido)-céph-3-em-4-carboxyliques de formule générale

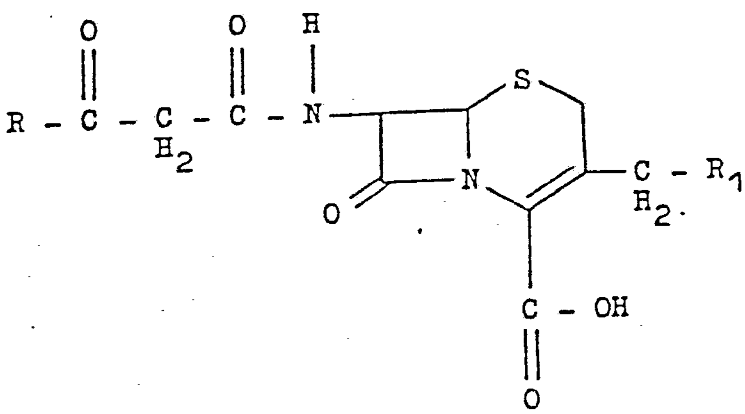

(V)

dans laquelle R et $R_1$ sont tels que définis plus haut, on nitrose ceux-ci de façon connue en soi, éventuellement sous forme protégée, avec des solutions de nitrites alcalins en présence d'acide acétique ou d'acide formique en vue d'obtenir des dérivés d'acides 7β-(cétoximinoacylamido)-céph-3-em-4-carboxyliques de formule I où $R_2$ représente de l'hydrogène, et

α) on alkyle ou acétyle ceux-ci éventuellement de façon connue en soi et/ou,

β) dans la mesure où ont été obtenus des dérivés d'acides 7β-(cétoximinoacylamido)-céph-3-em-4-carboxyliques de formule générale I où $R_1$ représente un reste acétoxy, on transforme éventuellement ceux-ci de façon connue en soi en les dérivés d'acides 7β-(cétoximinoacylamido)-céph-3-em-4-carboxyliques correspondants de formule générale I où $R_1$ représente un reste carbamoyloxy ou un reste de formule générale II, ou

b) on fait réagir de façon connue en soi des acides 3-oxo-2-alkoxyiminoalcaniques, respectivement des acides 3-oxo-2-acétoxyiminoalcaniques de formule générale

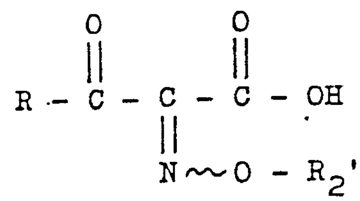

(VI)

dans laquelle $R_2'$ représente un reste alkyle avec 1 à 4 atomes de carbone ou un reste acétyle et R est tel que défini plus haut ou leurs dérivés fonctionnels réactifs, avec des acides 7β-aminocéphalosporanniques ou leurs dérivés de formule générale

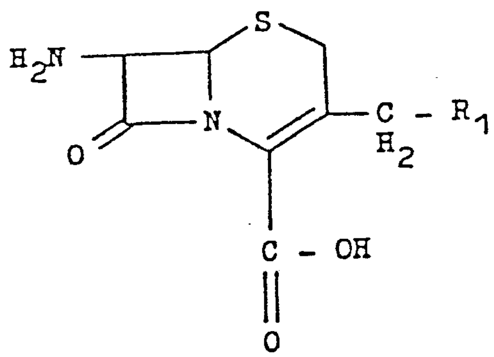

(III)

dans laquelle $R_1$ est tel que défini plus haut, sous forme protégée en vue d'obtenir des dérivés d'acides 7β-(cétoximinoacylamido)-céph-3-em-4-carboxyliques de formule générale I dans laquelle $R_2$ désigne un reste alkyle avec 1 à 4 atomes de carbone ou un reste acétyle, après quoi on élimine de façon connue en soi les groupes protecteurs éventuellement présents et/ou transforme éventuellement de façon connue en soi les dérivés d'acides 7β-(cétoximinoacylamido)-céph-3-em-4-carboxyliques obtenus de formule générale I, en des sels alcalins.

2. Procédé selon la revendication 1, caractérisé en ce qu'on prépare comme dérivés d'acides 7β-(cétoximinoacylamido)-céph-3-em-4-carboxyliques tels dans lesquels le reste alkyle qui peut être représenté par R est un reste avec 2 ou 3 atomes de carbone.

3. Procédé selon la revendication 1, caractérisé en ce qu'on prépare comme dérivés d'acides 7β-(cétoximinoacylamido)-céph-3-em-4-carboxyliques tels dans lesquels le reste hétérocyclique, qui peut être représenté par R, est un reste furyle, un reste thiényle ou un reste thiazolyle, éventuellement aminosubstitué.

4. Procédé selon les revendications 1 à 3, caractérisé en ce qu'on prépare comme dérivés d'acides 7β-(cétoximinoacylamido)-céph-3-em-4-carboxyliques tels dans lesquels le reste hétérocyclique représenté par Het est un reste tétrazolyle, thiazolyle, thiadiazolyle ou pyridazinyle.

5. Procédé selon les revendications 1 à 4, caractérisé en ce qu'on prépare comme dérivés d'acides 7β-(cétoximinoacylamido)-céph-3-em-4-carboxyliques tels dans lesquels le reste alkyle, respectivement le reste sulfoalkyle par lequel peut être substitué le reste hétérocyclique représenté par Het, est un reste avec 1 à 3, en particulier avec 1 ou 2 atomes de carbone.

6. Procédé selon les revendications 1 à 5, caractérisé en ce qu'on prépare comme dérivés d'acides 7β-(cétoximinoacylamido)-céph-3-em-4-carboxyliques tels dans lesquels le reste alkyle qui peut être représenté par $R_2$ est un reste avec 1 ou 2 atomes de carbone.

7. Procédé selon la revendication 1 ou 2, caractérisé en ce qu'on prépare l'acide 7β-(2'-hydroxyimino-3'-oxovaléramido)-3-(acétoxyméthyl)-céph-3-em-4-carboxylique.

8. Procédé selon la revendication 1 ou 2, caractérisé en ce qu'on prépare l'acide 7β-(2'-hydroxyimino-3'-oxo-4'-méthylvaléramido)-3-(acétoxyméthyl)-céph-3-em-4-carboxylique.

9. Procédé selon la revendication 1, 4 ou 5, caractérisé en ce qu'on prépare l'acide 7β-(2'-hydroxyimino-3'-oxovaléramido)-3-[(1''-méthyl-1''H-tétrazol-5''-yl)-thiométhyl]-céph-3-em-4-carboxylique.